(19) Europäisches Patentamt
European Patent Office
Office européen des brevets

(11) EP 2 125 029 B1

(12) EUROPEAN PATENT SPECIFICATION

(45) Date of publication and mention of the grant of the patent:
**12.10.2016 Bulletin 2016/41**

(21) Application number: **07856911.8**

(22) Date of filing: **19.12.2007**

(51) Int Cl.:
***A61K 47/48*** *(2006.01)* ***C07H 21/00*** *(2006.01)*
***A61P 35/00*** *(2006.01)* ***A61P 43/00*** *(2006.01)*
***A61K 9/127*** *(2006.01)*

(86) International application number:
**PCT/EP2007/011188**

(87) International publication number:
**WO 2008/074488 (26.06.2008 Gazette 2008/26)**

(54) **CONSTRUCTION AND USE OF TRANSFECTION ENHANCER ELEMENTS**

KONSTRUKTION UND VERWENDUNG VON TRANSFEKTIONS-VERSTÄRKUNGSELEMENTEN

CONSTRUCTION ET UTILISATION D'ÉLÉMENTS AMÉLIORANT LA TRANSFECTION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **19.12.2006 EP 06077234**

(43) Date of publication of application:
**02.12.2009 Bulletin 2009/49**

(73) Proprietor: **Lipocalyx GmbH**
**06766 Wolfen (DE)**

(72) Inventor: **PANZNER, Steffen**
**06114 Halle (DE)**

(74) Representative: **Blodig, Wolfgang**
**Wächtershäuser & Hartz**
**Patentanwaltspartnerschaft mbB**
**Weinstrasse 8**
**80333 München (DE)**

(56) References cited:

| | |
|---|---|
| **EP-A- 1 935 434** | **WO-A-00/59474** |
| **WO-A-01/51689** | **WO-A-93/15742** |
| **WO-A-96/05218** | **WO-A-96/07392** |
| **WO-A-97/40854** | **WO-A-98/32467** |
| **WO-A-2006/053646** | **WO-A-2008/074487** |
| **US-A1- 2004 116 687** | **US-A1- 2005 164 963** |
| **US-B1- 6 989 434** | |

- **HENRY SCOTT M ET AL: "pH-responsive poly(styrene-alt-maleic anhydride) alkylamide copolymers for intracellular drug delivery" BIOMACROMOLECULES, vol. 7, no. 8, August 2006 (2006-08), pages 2407-2414, XP002454257 ISSN: 1525-7797**
- **CHEUNG CHARLES Y ET AL: "A pH-sensitive polymer that enhances cationic lipid-mediated gene transfer" BIOCONJUGATE CHEMISTRY, vol. 12, no. 6, November 2001 (2001-11), pages 906-910, XP002454175 ISSN: 1043-1802**
- **FATTAL E ET AL: ""Smart" delivery of antisense oligonucleotides by anionic pH-sensitive liposomes" ADVANCED DRUG DELIVERY REVIEWS 23 APR 2004 NETHERLANDS, vol. 56, no. 7, 23 April 2004 (2004-04-23), pages 931-946, XP002454176 ISSN: 0169-409X**
- **MATRAY TRACY J ET AL: "Synthesis of oligonucleotides containing 3'-alkylcarboxylic acids using a palladium labile oligonucleotide solid phase synthesis support" BIOCONJUGATE CHEMISTRY, vol. 8, no. 2, 1997, pages 99-102, XP002504282 ISSN: 1043-1802**
- **REMY J-S ET AL: "GENE TRANSFER WITH A SERIES OF LIPOPHILIC DNA-BINDING MOLECULES" BIOCONJUGATE CHEMISTRY, ACS, WASHINGTON, DC, US, vol. 5, no. 6, 1 November 1994 (1994-11-01), pages 647-654, XP000484178 ISSN: 1043-1802**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 125 029 B1

- ZHOU X ET AL: "LIPOPHILIC POLYLYSINES MEDIATE EFFICIENT DNA TRANSFECTION IN MAMMALIAN CELLS" BIOCHIMICA ET BIOPHYSICA ACTA. BIOMEMBRANES, AMSTERDAM, NL, vol. 1065, no. 1, 31 May 1991 (1991-05-31), pages 8-14, XP000197616 ISSN: 0005-2736
- SOCHANIK ALEKSANDER ET AL: "In vivo gene transfer using cetylated polyethylenimine" ACTA BIOCHIMICA POLONICA, vol. 51, no. 3, 2004, pages 693-702, XP002511892 ISSN: 0001-527X
- POTIER P ET AL: "Synthesis and hybridization properties of oligonucleotides containing polyamines at the C-2 position of purines: a pre-synthetic approach for the incorporation of spermine into oligodeoxynucleotides containing 2-(4,9,13-triazatridecyl)-2'-deoxyguanosin e" CHEMISTRY - A EUROPEAN JOURNAL, WILEY - V C H VERLAG GMBH & CO. KGAA, WEINHEIM, DE, vol. 6, no. 22, 17 November 2000 (2000-11-17), pages 4188-4194, XP002454258 ISSN: 0947-6539
- TAKEDA TADAYUKI ET AL: "NUCLEOSIDES AND NUCLEOTIDES. LXXVI. SYNTHESIS AND PROPERTIES OF DEOXYOLIGONUCLEOTIDES CONTAINING PUTRESCINYLTHYMINE" CHEMICAL AND PHARMACEUTICAL BULLETIN, PHARMACEUTICAL SOCIETY OF JAPAN, TOKYO, vol. 35, no. 9, 1 January 1987 (1987-01-01), pages 3558-3567, XP001248435 ISSN: 0009-2363
- RAMASAMY K S ET AL: "REMARKABLE ENHANCEMENT OF BINDING AFFINITY OF HETEROCYCLE-MODIFIED DNA TO DNA AND RNA. SYNTHESIS, CHARACTERIZATION AND BIOPHYSICAL EVALUATION ON N2-IMIDAZOLYLPROPYLGUANINE AND N2-IMIDAZOLYLPROPYL-2AMINOADENINE MODIFIED OLIGONUCLEOTIDES" TETRAHEDRON LETTERS, ELSEVIER, AMSTERDAM, vol. 35, no. 2, 10 January 1994 (1994-01-10), pages 215-218, XP002052846 ISSN: 0040-4039
- HASHIMOTO HIROMASA ET AL: "Zwitterionic DNA" JOURNAL OF THE AMERICAN CHEMICAL SOCIETY, vol. 115, no. 16, 1993, pages 7128-7134, XP002512015 ISSN: 0002-7863
- FRALEY A W ET AL: "Cationic oligonucleotide-peptide conjugates with aggregating properties enter efficiently into cells while maintaining hybridization properties and enzymatic recognition" JOURNAL OF THE AMERICAN CHEMICAL SOCIETY 20060823 US, vol. 128, no. 33, 23 August 2006 (2006-08-23), pages 10763-10771, XP002512016 ISSN: 0002-7863
- SANEYOSHI H ET AL: "A General Method for the Synthesis of 2'-O-Cyanoethylated Oligoribonucleotides Having Promising Hybridization Affinity for DNA and RNA and Enhanced Nuclease Resistance" JOURNAL OF ORGANIC CHEMISTRY, AMERICAN CHEMICAL SOCIETY, EASTON.; US, vol. 70, no. 25, 1 January 2005 (2005-01-01), pages 10453-10460, XP003017305 ISSN: 0022-3263


Remarks:

The file contains technical information submitted after the application was filed and not included in this specification

## Description

### Field of the invention

This disclosure describes structural elements that enable transport of otherwise impermeable polar substances across biological membranes, in particular cell membranes. The elements are pH sensitive in terms of charge and hydrophilicity and undergo a polar - apolar transition when exposed to low pH.

### Background of the invention

Biological cells are surrounded and sealed with a continuous membrane which is impermeable to polar solutes irrespective of their molecular dimension. Although very small molecules such as water or urea are still able to penetrate a lipid bilayer, diffusion of charged ions is already very slow and the membrane is practically impermeable for somewhat bigger polar molecules, e.g. glucose or calcein. Independent from size, hydrophilicity is the second big factor that has an impact on the ability of a molecule to penetrate lipid bilayers and molecules showing some solubility in both the watery phase and the lipid phase can cross lipid bilayers and equilibrate between both sides of the membrane, e.g. the interior and exterior of a cell or the aqueous inner volume and the external buffer phase of a liposome. This basic understanding is guiding the synthesis and optimization of small molecule drugs and is part of the "Lipinski rule of five" (Lipinski et al., Advanced Drug Delivery Reviews, 23, 3-25, 1997). Hydrophilicity is expressed as logP, the equilibrium constant of a given solute between 1-octanol and water; values >1 indicate a preferential distribution into the apolar phase of 1-octanol, values <1 indicate better solubility in water than 1-octanol.

In many cases the molecules of interest comprise protonable groups of either acidic or basic character and the state of protonation has an impact on the hydrophilicity of the molecule. A common descriptor that reflects pH dependent hydrophilicity is logD, which is the logP at a given pH.

The knowledge for such changes in the distribution is widely used in chemical synthesis and purification, e.g. when a weakly basic molecule is re-distributed from a water phase into the organic phase of a two phase system by raising the pH of the solution.

More specifically, such understanding is fundamental for practicing the remote loading of liposomes with small pH-sensitive solutes as disclosed in Madden et al. , Chem. Phys. Lipids, 53(1), 37-46, 1990 or Harrigan et al. , Biochim. Biophys. Acta, 1149(2), 329-338, 1993, amongst others. Drugs like doxorubicin are provided in a buffer of neutral or slightly basic pH at or above the pka of the molecule. The unprotonated form is membrane permeable and equilibrates between the bulk solution and the interior of added liposomes. Now, if such liposomes contain a buffer of low pH, the drug molecule becomes protonated and cannot escape the liposome anymore. The process continues until the buffer capacity of the liposome interior is exhausted or all drug resides within the liposome and distributions far from equilibrium can be reached.

The same principle can be applied to weak acids, e.g. the carboxylic acids which are soluble or miscible with organic solvents in their undissociated form but not in the salt form.

Penetration of carboxylic acids through lipid bilayers has been reported e.g. by Clercs et al., Biochim. Biophys. Acta, 1240(2), 257-265, 1995.

A particular field of this invention is the transport of nucleic acids and more specifically the transport of oligonucleotides across biological membranes. Penetration of such molecules is hampered by their very hydrophilic and charged nature and efforts have been made to reduce the hydrophilic nature of such molecules by means. Chemical modification of the internucleoside linkage can eliminate the charged character of the phosphodiester bond, e.g. by using methylphosphonates (Miller and Ts'o 1981,Annu Rep Med Chem 23:295) or can reduce it through incorporation of phosphorothioate bonds (Eckstein 1989, Trends Biochem Sci 14:97) or phosphorodithioate bonds (Nielsen 1988, Tetrahedron Lett 29:2911). Rudolph et al. (1996 in Nucleosides and Nucleotides 15:1725) introduced phosphonoacetate derivatives of oligonucleotides and Dellinger in US 6,693,187 and its continuations US 7,067,641; US2004/0116687 and US2006/0293511 presents further data on synthesis of such compounds. Phosphonoacetates were profiled as derivatives of oligonucleotides with reduced internucleoside charge that are highly nuclease resistant and, when designed as single stranded oligodeoxynucleotides, facilitate catalytic action of RNAseH upon binding to a complementary strand of RNA (in Sheehan et al, Nucl Acid Res 2003, 31:4109-4118). The thymidine dimers presented there display a decreased hydrophilicity at low pH; however, the cellular uptake of an oligonucleotide remained unchanged. In fact, cellular penetration was only achieved after elimination of the carboxylate charge group by esterification with methyl- or butyl groups.

In still other cases, lipophilic conjugation has been used to improve the cellular uptake of oligonucleotides such as single stranded oligodeoxynucleotides or double stranded siRNA molecules (Letsinger et al. in US 4958013 or Proc. Natl. Acad. Sci., 86, 6553-6556, 1989 or by Manoharan et al. in US 6,153,737 and US 6,753,423 in combination with single stranded oligonucleotides; Soutschek et al.(2004) Nature, 432(7014), 173-178 or Wolfrum et al.(2007) in Nat Biotech 25:1149-1157 for the delivery of siRNA.

US 6,989,434 B1 discloses an element used for transfection comprising spermine and a steroid group.

### Objectives of the invention

The penetration problem of oligonucleotides remains challengig today and alternative approaches for the problem of cellular uptake still represent a major technical need for this class of substances.
It is therefore an object of this invention to provide oligonucleotides or their designs with improved cellular penetration.
A mere minimization of the hydrophilic character through the conjugation of hydrophobic elements such as cholesterol or long chain alkyl groups was shown to enhance uptake in vivo, but was also correlated to improved binding of such conjugates to LDL particles and related improvements in retention and biodistribution (Wolfrum et al. (2007); Nat Biotech 25:1149-1157.
It is therefore a further objective of the invention to provide oligonucleotides or their designs wherein such minimization of hydrophilic character can be triggered by external stimuli, while keeping polarity of the molecules high upon storage and administration.
It was surprisingly found that pH sensitive moieties selected from the group comprising weak acids, or zwitterions having a cationic charge that is protonable at slightly acidic pH, in combination with bydrophobic moieties can mediate the required change in hydrophilicity.
This invention makes use of pH-sensitive hydrophilicity elements, so called transfection enhancer elements or TEE's within this disclosure, capable of transporting polar molecules across membranes.
This disclosure describes combinations of TEE's s with nucleic acids. In addition, this disclosure provides guidance on the design of novel oligonucleotides and optimization of such designs to improve membrane permeability of this class of drugs.
Also described is an application of the teachings of the present invention to the cellular penetration of other polar entities such as peptides or proteins or carrier systems sequestering the active ingredients, or small molecules, sugars or other polar molecules from different origin.

### Brief description of the invention

Accordingly, this invention provides a complex comprising a nucleic acid and a carrier system having grafted thereon one or more transfection enhancer elements (TEE's), as defined in claim 1.
The invention also provides a polycation having grafted thereon TEE's, as defined in claim 6.
The invention further provides a nucleic acid comprising a nucleic acid moiety and one two or more TEE's, as defined in claim 8.
Also described are conjugates of nucleic acids with two or more pH-responsive transfection enhancer elements (TEE's) with the general structure (I)
Hydrophobic element - pH-responsive hydrophilic elements (I).
The position of the hydrophilic element within the TEE structure may vary. In some aspects, the hydrophilic element is located distal from the link between molecule and TEE. In other aspects, the hydrophilic element is located central within the TEE.
The pH-responsive hydrophilic element comprises weak acids having a pKa of between 2 and 6, preferred of between 3 and 5. Herein, said weak acids are carboxyl groups.
In other embodiments said pH-responsive hydrophilic element may be a zwitterionic structure comprising a combination of weak or strong acidic groups with weak bases, the latter having a pka of between 3 and 8, preferred of between 4.5 and 7. Suitably said zwitterionic structures may be formed from an anionic group and a heterocyclic nitrogen atom as cationic group. To achieve specific pKa's s of said hydrophilic elements, said pH-responsive hydrophilic element may be substituted with structural elements, selected from the group comprising hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chlorid-, chlormethyl- vinyl-, phenyl-, benzyl-, methyl-, ethyl- , propyl-, isopropyl- and tert-butyl or cyclohexyl groups.
Said hydrophobic elements are linear, branched or cyclic chains having between 6 and 40 units. In one aspect of this embodiment said hydrophobic element comprises more than 6 and up to 40 units, in a second aspect said hydrophobic element comprises between 6 and 20 units and in a third aspect said hydrophobic element comprises between 20 and 40 units.
The units of said hydrophobic element are carbon atoms. In one embodiment said hydrophobic element can be saturated or may contain unsaturated bonds. In other embodiments said hydrophobic element or its units may be substituted.
In some embodiments the branching of the main chain of said hydrophobic element may comprise rather small

building blocks. Preferred building blocks comprise methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.
Alternatively, said hydrophobic element may derive from sterols, said sterols may be further substituted.

It is possible to insert one or more heteroatoms or chemical groups into the hydrophobic element of the pH-responsive transfection enhancer elements (TEE's). Such heteroatoms or chemical groups may be selected from -O-, -S-, -N(H)C(O)-, -C(O)O-, -OC(O)N(H)-, -C(O)-, -C(O)-N(H)-, -N(H)-C(O)-O-,-CH=N-, -O-C(O)-, -N=CH- and/or -S-S-, amino acids or derivatives thereof, $\alpha$-hydroxyacids or $\beta$-hydroxy acids.

TEE's undergo a hydrophile-hydrophobe transition in response to an acidification of the environment. This transition is mediated by the hydrophilic elements described above that are responsive towards pH.

In preferred embodiments of the invention logD(4.0)-logD(7.4)>=1 for the transfection enhancer elements; the logD at pH 7,4 is between -2 and 10 and the logD at pH 4 of said pH-responsive transfection enhancer elements (TEE's) exceeds 0.

Of course, said pH-responsive transfection enhancer elements (TEE's) may contain more than one pH responsive hydrophilic element.

In particular aspects the pH-responsive transfection enhancer elements (TEE's) are grafted onto oligonucleotides via covalent chemical bonds. Such graft may sit at various positions including the 2' O- or 2' S-positions of the nucleosides, the nucleobase, the internucleoside linkages, e.g. the phosphate backbone or modifications thereof, the peptide backbone in peptide nucleic acids, the heterocyclic backbone in morpholino nucleic acids and the like. The graft may also be attached to the phosphoribose or phosphodexyribose backbone, replacing the former nucleobases at C1.

In some embodiments no more than 2/3 of all nucleobases of said oligonucleotide are modified with said pH-responsive transfection enhancer elements (TEE's).

In other embodiments only nucleobases at the flanks of said oligonucleotides are modified with pH-responsive transfection enhancer elements (TEE's) leading to gapmer structures.

In other preferred aspects, said oligonucleotides comprise polymeric or oligomeric extensions wherein the polymer backbone structure is not a phosphoribose and said extensions carry one or more TEE's. In some aspects such extension represents polyamines forming oligomeric or multimeric forms of TEE's, such as TEE's linked to polyamines.

Said pH-responsive transfection enhancer elements (TEE's) may be alkylcarboxylic acids preferably comprising between 8 to 16 carbon atoms. Alternatively, said pH-responsive transfection enhancer elements (TEE's) may comprise sterols.

It is also possible that said pH-responsive transfection enhancer elements are grafted onto polycationic elements that form a complex controlled in size with said nucleic acids.

In some embodiments said polycationic elements are polyamines selected from the group comprising polyethyleneimine, spermine, dispermine, trispermine, tetraspermine, oligospermine, thermine, spermidine, or putrescine.

In a particular embodiment of the present invention, said nucleic acids are oligonucleotides, polynucleotides or DNA plasmids.

Also described are pharmaceutical compositions comprising conjugates of nucleic acids with transfection enhancer elements (TEE's) as defined herein and a pharmaceutically acceptable vehicle therefor.

Further described is the use of a pharmaceutical composition for the treatment or prophylaxis of inflammatory, immune or autoimmune disorders and/or cancer of humans or non-human animals.

In another aspect, described is the use of pH responsive transfection enhancer elements (TEE's) for the in vivo, in vitro or ex vivo transfection of polar solutes. The polar solutes may be selected from the group comprising small molecules, proteins, peptides, carbohydrates or nucleic acids.
One or more transfection enhancer elements (TEE' s) may be conjugated to said polar solutes by chemical bonds, physical attraction or by other interactions. Alternatively, said transfection enhancer elements (TEE's) may be conjugated to carrier systems sequestering said polar solutes.

For clarity, the following definitions and understandings are used for important terms of the invention:

LogP
...is the ratio of the respective concentrations of a compound in the 1-octanol and water partitions of a two-phase system at equilibrium. The octanol-water partition coefficient (logP) is used to describe the lipophilic or hydrophobic properties of a compound.

LogD
...is the ratio of the equilibrium concentrations of all species (unionized and ionized) of a molecule in 1-octanol to same species in the water phase. The partition coefficient for dissociative mixtures, logD, is defined as follows:

$$\log D = \log\left(\Sigma(c_i^{H2O})/\Sigma(c_i^{org})\right),$$

where

$c_i^{H2O}$ is the concentration of the i-th microspecies in water and
$c_i^{org}$ is the concentration of the i-th microspecies in the organic phase.

LogD differs from logP in that ionized species are considered as well as the neutral form of the molecule. LogD is therefore the logP at a given pH of the medium.

LogP and logD values can be determined experimentally by measuring the partition of a molecule or its ionized forms in octanol/water systems. Experimental values have been generated for a vast amount of individual compounds and expert systems allow extrapolating logP and logD values for novel species. One such expert system is ACD/Labs with the modules ACD/LogP or ACD/logD and ACD/Labs 7.06 has been used for calculations within this disclosure.

"Nucleic acid" or "Polynucleotide" .. as used herein refers to any nucleic acid containing molecule, including without limitation, DNA or RNA. The term polynucleotide(s) generally refers to any polyribonucleotide or polydeoxyribonucleotide, which may be unmodified RNA or DNA or modified RNA or DNA. Thus, for instance, polynucleotides as used herein refers to, among others, single-and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions.

Oligonucleotide

... as used herein is defined as a molecule with two or more deoxyribonucleotides or ribonucleotides, often more than three, and usually more than ten. The exact size of an oligonucleotide will depend on many factors, including the ultimate function or use of the oligonucleotide. Oligonucleotides can be prepared by any suitable method, including, for example, cloning and restriction of appropriate sequences and direct chemical synthesis by a method such as the phosphotriester method of Narang et al., Meth. Enzymol., 68, 90-99, 1979; the phosphodiester method of Brown et al., Method Enzymol., 68, 109-151, 1979 the diethylphosphoramidite method of Beaucage et al., Tetrahedron Lett., 22, 1859-1862, 1981 the triester method of Matteucci et al., J. Am. Chem. Soc., 103, 3185-3191, 1981 or automated synthesis methods; and the solid support method of US 4,458,066.

Transfection

...is used widely to specifically describe the disappearance of a concentration gradient across a biological membrane in vivo, in vitro or ex-vivo. It comprises transport across, or diffusion through, penetration or permeation of biological membranes irrespective of the actual mechanism by which said processes occur.

## Detailed description of the invention

Transfection enhancer elements are pH-responsive amphiphiles with the general structure:

(I) hydrophobic element - pH-responsive hydrophilic element

The pH-sensitive hydrophilicity elements are called transfection enhancer elements or TEE's within this disclosure.

In some cases, a combination of short alkylcarboxylic acids with oligonucleotides has been mentioned. Rudolph et al. (1996 in Nucleosides and Nucleotides 15:1725) introduced phosphonoacetate derivatives of oligonucleotides and Dellinger in US 6,693,187 and its continuations US 7,067,641; US2004/0116687 and US2006/0293511 presents further data on synthesis of phosphonoacetaes, phosphonoformiates and related compounds. Phosphonoacetates or phosphonoformiates share the pH sensitive element of a TEE, but fail to provide sufficient length for the hydrophobic segment. In comparison to a phosphodiester bond its phosphonoacetate or phosphonoformiate derivatives are even more hydrophilic at pH7.4 ($\Delta$ logD= - 0,2 and -0,4 per internucleoside bond) and any decrease in this hydrophilicity at pH4 is modest ($\Delta$ logD= +0,3 and 0 for phosphonoacetate or -formiate, respectively).

Oligonucleotides that comprise alkyl moieties optionally terminated by carboxyl groups can also be found in US 6277967 (Guzaev and Manoharan) or US 6919437 (Manoharan), but alkylcarboxylic acids were mentioned inter

alia and no specific disclosure on the selection of such modifications or use of such modifications for the improvement of membrane penetration was made in there.

Prior art is silent to the selection and optimization of structural elements (I) to enhance cellular uptake and cytosolic delivery of oligonucleotides. Prior art has not taught, that the combination of hydrophobic elements with the pH sensitive hydrophilic elements provides criticality to such function. In some cases, the prior art suggests esterification of the charge responsive element in order to facilitate cellular uptake (Sheehan 2003, Nuc Acid Res 31: 4109-4118) followed by an enzymatic hydrolysis that re-instates the activity of the compound. This is contrary to the teachings of the invention described here.

Hydrophilic elements of the TEE's

The hydrophilic elements are weak acids that provide a response in hydrophilicity between pH values of about 4 and the physiological pH of 7.4.

Carboxyl groups are used according to this invention, see formula (1) in table 1.

Table 1: Compound 1

| | |
|---|---|
| (1) 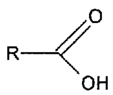 | (1) Carboxylic acids. R represents the hydrophobic element of the invention. |

LogD values for hydrophilic head groups derived from (1) are high at low pH and low at neutral or higher pH.

In another aspect of the invention, the hydrophilic elements comprise zwitterionic groups that respond to changes in the pH of the environment. Zwitterionic structures exist at pH values where both the cationic and the anionic group are charged and a generalized logD plot is shown in figure 1. It is apparent that the zwitterions have higher logD values than the charged parent groups and the maximum amplitude in logD for the zwitterion formation is about 2.5 units.

The desired increase in logD upon acidification is represented by the right flank of the logD curve and depends on the pKa of the cationic charge group; it is rather independent from the pKa of the anionic group itself. As an example, the anionic group maybe a carboxyl group and the cationic group maybe a heterocyclic nitrogen atom two to five carbon atoms apart from that group (e.g. compounds 10 or 11). Pyridylcarboxylic acids, imidazolcarboxylic acids or the like are a few representations of such pH-responsive hydrophilic elements. The zwitterion exists between pH 4 and pH 7, thereby providing the pH-responsive hydrophilic headgroups of the invention. On the contrary, a simple amino group having a high pKa of about 9 (e.g. compound 13) or a quarternary ammonium group providing a constant positive charge with no effective pKa (e.g. compound 12) extends the range of pH values where the zwitterion exists and any change in hydrophilicity does no longer occur in the pH region desired (pH 2 to 9 or the more preferred ranges given above, see figure 1 and table 2). It becomes apparent that the guiding pKa for the zwitterion is the pKa of the basic counterpart. In preferred embodiments, the pKa for a base in zwitterions is between 3 and 8, preferred between 4.5 and 7 to achieve a pH dependent response in logD between pH 4 and 7.4.

Table 2: Compounds 10-14

| | | |
|---|---|---|
| (10) 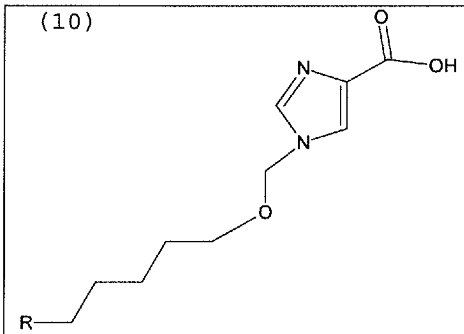 | (12) 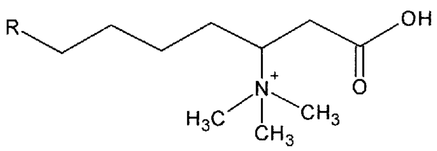 | (11) 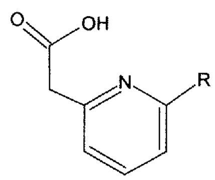 |
| (13) 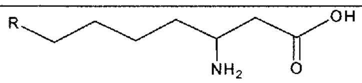 | (14) 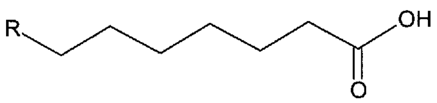 | |

The hydrophilic elements can further be substituted with polar or apolar groups. Substitutions may be selected to achieve a specific pKa of the hydrophilic element. Rules to achieve such adjustment of pKa values are known to the skilled artisan and comprise for example substitutions at nitrogen atoms of barbituric acid or xanthine with hydroxymethyl-, hydroxyethyl-, methoxymethyl-, methoxyethyl-, ethoxymethyl-, ethoxyethyl-, thiomethyl-, thioethyl-, methylthiomethyl-, methylthioethyl-, ethylthiomethyl-, ethylthioethyl-, chlorid-, chlormethyl- vinyl-, phenyl- or benzyl groups or mixtures thereof to achieve a lower pKa of the structure. Substitutions at the positions R1, R2 or R3 in formula (2) or (3) are in particular suitable to achieve such shift in pKa values.

Of course, pKa values can be shifted towards higher values with substitutents comprising methyl-, ethyl- , propyl-, isopropyl- and tert-butyl or cyclohexyl groups or mixtures therof.

It is known, that the pKa value for carboxyl groups is also affected by substitutions or chemical alterations in spatial proximity. Acrylic acid derivatives, aromatic carboxylic acids such as benzoic acid, pyridinyl carboxylic acid, α- or β- hydroxycarboxylic acids or α- or β-thiocarboxylic acids but also halogenated carboxylic acids have lower pKa values than the parent compounds. In contrast, substitutions with an +I effect change the pKa of a carboxyl group towards higher values, e.g. in cyclohexylcarboxylic acids.

Specific examples of substituted hydrophilic elements include, but are not limited to formula (7), (8) and (9) of table 3, wherein R identifies the hydrophobic element of the TEE:

Table 3: Compounds 7-9

| Substituted carboxylic acids | | |
|---|---|---|
| (7) 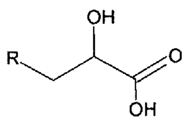 | (8) 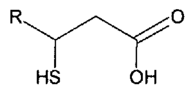 | 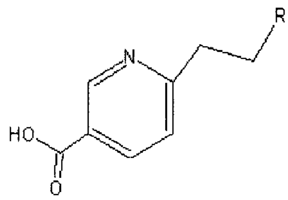 (9) |

Chemical representations for the hydrophilic elements can be identified from the group of weak acids using the relationship between logD, pH and the pKa of the substance. For acids, this can be expressed as follows:

$$logD = logP + log( 1 + 10^{(pH-pKa)} );$$

wherein logP is the partition coefficient for the non-ionized form. The equation reflects conditions of zero ionic strenght and extremely low values for logD are calculated for acids at high pH. Under physiological conditions, where the ionic strenght is about 0,15M, salt formation is limiting such extremes in logD.

Figure 2 shows the logD calculations for a number of hydrophilic elements.

Further analysis reveals identical shifts in logD when curves are plotted against pH-pKa (see figure 3).

Once standardized with respect to their pKa values the logD plots become similar for all hydrophilic elements analyzed here. A maximum difference of 3.75 units in logD can be achieved for the ionization of a single hydrophilic element. The maximum amplitude for zwitterion formation is about 2.5 units in logD.

The full amplitude requires a rather large shift of about 6 units in pH. Within the practical range of ΔpH ~ 3.4 (pH 7.4 - pH 4) considered for many aspects of this invention, the maximum difference in logD is about 3 units for pKa ~ 4. For a single charged element, the logD reacts sensitive whenever the pH is 0 to 4 units above the pKa, the most sensitive reaction is at pH values between 1 and 2.5 units above pKa. However, when multiple TEE's are used, e.g. a number of TEE's grafted onto the backbone of an oligonucleotide, the logD/pH curves become increasingly steep. Such lateral compression of logD responses is analyzed in figure 4 for methylene bridged multimers of decanoic acid. While decanoic acid per se shows an extended response between pH 4.5 and 9, the right flank of such a response shifts towards lower pH values upon multimerization of the TEE. A decamer of decanoic acid reacts in a narrow range between pH 4.5 and 6,25.

In practical terms, the ideal pKa for hydrophilic elements is about 4. Preferred are hydrophilic elements having a pKa between 2 and 6 (maximum amplitude about 1.5 units), more preferred are hydrophilic elements having a pKa between 3 and 5 (maximum amplitude about 2.5 units). Other hydrophilic elements within the scope of the invention may have pKa values between 1 and 7. For use with multimeric structures such as oligonucleotides the optimal pKa of the hydrophilic element is higher and needs to be more precisely targeted towards the intended response range. The most preferred pKa of TEE's for oligonucleotides is 4.5 to 5.5. Still preferred are pK values between 4 and 6.5.

In preferred embodiments the pH sensitive hydrophilic moiety is a weak acid of the following general formula (II) having

a pka of between 1 and 7, preferred between 2 and 6 and more preferred between 3 and 5:

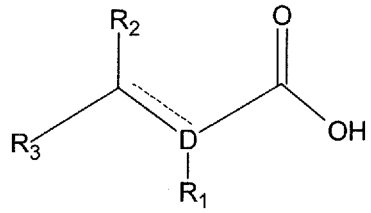

(II)

wherein the dotted line represents a double bond which is optional and R1, R2, and R3 are independently the hydrophobic moiety of the TEE, hydrogen, linear, branched or cyclic, unsubstituted or substituted $C_1$-$C_{10}$ alkyl, alkylene or heteroalkyl having 0-5 sites of unsaturation, or aryl group and said groups comprising 0-5 heteroatoms, selected from -O- or -S-, wherein said heteroatoms are not the first atom in said groups and wherein said substituents are selected from hydroxy-, mercapto, oxo-, formyl-, nitro-, cyano-, halo- or a trihalomethyl group and wherein R1, R2, and R3 may be alternatively and independently alkoxy-, alkoxyalkyl-, alkylthio-, alkylthioalkyl-, hydroxy-, mercapto-, oxo-, formyl-, nitro-, cyano-, halo- or a trihalomethyl group and D is C or an unsubstituted or substituted cyclic alkyl or aryl group having 0-3 sites of unsaturation and comprising 0-5 heteroatoms selected from -O-, -S-, and said substituents are selected from alkyl-, alkylene-, alkenyl-, alkynyl-, alkoxy-, alkoxyalkyl-, alkylthio-, alkylthioalkyl-, hydroxy-, mercapto-, oxo-, formyl-, cyano-, halo- or a trihalomethyl group.

Also described are cases wherein said pH sensitive hydrophilic element of the TEE forms

(VI)

a zwitterion upon acidification, wherein the cationic charge of said zwitterion is preferably a nitrogen atom of a weak base having a pka of between 3 and 8, preferred between 4.5 and 7 and more preferred between 5 and 6.5. In one aspect said weak base is imidazole, morpholine, pyridine or piperazine. Alternatively, said weak base is a non-cyclic amine.

It is known in the art that the pKa of such weak bases can be affected by heteroatoms in β- or γ-position of the nitrogen atom or by substitutions in spatial proximity, for example in α- or β-position of the nitrogen atom. Prefered heteroatoms are -O- or -S-. Substituents with an -I effect are capable for lowering the pka of such weak bases and include without limitation alkoxy, alkylthio, hydroxy-, mercapto-, oxo-, formyl-, nitro-, cyano-, halo- or trihalomethyl groups. Substituents with an +I effect are capable for increasing the pka of such weak bases and include for example alkyl groups. The heteroatoms in β- or γ-position of the nitrogen atom and the substituents in α- or β-position of the nitrogen atom may be part of the hydrophobic element of the TEE. The anionic charge of said zwitterion is an acidic group selected from carboxyl-, phosphate-, phophite-, sulfo- or sulfino groups. Prefered are carboxyl groups. The anionic charge may be between 2 and 5 carbon atoms apart from the cationic charge of the zwitterion.

Hydrophobic elements of the TEE's

TEE's of this invention comprise hydrophobic elements which contribute to the penetration of biological lipid membranes. Chemical representations of such hydrophobic elements are linear, branched or cyclic chains with a minimum chain length of 6 units. The units are carbon atoms. In some aspects of the invention the units may comprise heteroatoms being able to form covalent bonds with more than one other chain element. Hydrogen or halogen atoms can substitute the chain, but are not units by themself. The hydrophobic elements can comprise more than 6 units and may comprise up to 40 units and in selected aspects they comprise only 6 units. In some aspects of the invention the hydrophobic elements comprise between 6 and 12 units. In other aspects of the invention the hydrophobic element comprise between 12 and 20 untis. In still other aspects of the invention the hydrophobic element does comprise between 20 and 40 units.

In one aspect, the branching of the main chain comprises one or more rather small building blocks such as methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof.

Hydrophobic elements can be saturated or may contain unsaturated bonds.

In another aspect, more complex branched and or cyclic ring systems may be chemical representations of the hydrophobic element. In one embodiment of such aspect, hydrophobic elements are derived from sterols. Of course, the sterols may further be substituted with methyl-, ethyl-, propyl-, isopropyl-, methoxy-, ethoxy-, methoxymethyl-, ethoxymethyl-, methoxyethyl-, ethoxyethyl- and vinyl- or halogen groups or mixtures thereof. In another aspect of the invention, the hydrophobic elements may comprise sterols that are substituted with one or more hydrophilic groups such as hydroxyl groups. In a preferred embodiment, sterols contain hydroxyl groups at one or more of the positions 3, 7 and 12. In another aspect, the sterol is a cholestan and in a preferred embodiment the cholestan is hydroxylated in one or more of the positions 3, 7 or 12.

It is possible to insert one or more heteroatoms or chemical groups into the hydrophobic element. In one embodiment of the invention said hydrophobic element comprises no more than 5 and in another embodiment no more than 2 heteroatoms or chemical groups.

The heteroatoms or chemical groups may be selected from the group comprising -O-, -S-, -N(H)C(O)-, -C(O)O-, -OC(O)N(H)-, -C(O)-, -C(O)-N(H)-, -N(H)-C(O)-O-, -CH=N-, -O-C(O)-, -N=CH- and/or -S-S-.

Alternatively, said heteroatoms and/or chemical groups derive from amino acids, α-hydroxyacids or β-hydroxy acids.

In one embodiment said amino acid building block is selected from the group of proline, glycin, alanine, leucine, isoleucine, valin, tyrosine, tryptophane, phenylalanine or methionine or peptides thereof and said α-and β-hydroxyacids are selected from the group comprising glycolic acid, lactic acid or hydroxybutyric acid.

In another embodiment of such an aspect, more than a single ether group is present and the spacing between the ether bonds is two carbon atoms, representing the monomer elements in poly-ethylenglycol or polypropylenglycol.

Architecture of TEE's

Construction of TEE's is governed by its physicochemical parameters; TEE's undergo a hydrophile-hydrophobe transition in response to an acidification of the environment. This transition is mediated by the hydrophilic elements described above that are responsive towards pH. Such response should have minimum amplitude of 0.5 logD whereby the higher absolute value of logD is achieved at the lower pH. In one aspect, such amplitude is higher than 1 logD which means that a distribution of such TEE from the water phase to the hydrophobic interior of the membrane becomes 10 times more preferred. In another aspect, such amplitude is higher than 1.5 and in some aspects the amplitude between the hydrophilic and hydrophobic form is more than 2 logD units.

The hydrophilic elements can respond to pH values between the physiological pH and slightly acidic conditions of about pH 4. Such slightly acidic conditions can be found within cell organelles like endosomes or lysosomes. Therefore TEE's may be capable of mediating the endosomal escape of drugs after endocytotic uptake into the cell. Tumor tissue or areas of ongoing inflammation also provide a slightly acidic environment and consequently TEE's may be useful to accumulate drugs in these areas. Accumulation may occur specifically in tumor or stroma cells or in cells of the immune system or fibroblasts that are present in inflammatory regions. In a preferred embodiments the pK of the TEE is be optimized to maximize the difference between hydrophilicity at pH7.4 and hydrophobicity at pH4. In preferred aspects, the pKa of the hydrophilic elements essentially driven by weak acids is between 2 and 6. In more preferred aspects this pka is between 3 and 5. For use with multimeric structures such as oligonucleotides the optimal pKa of the hydrophilic element is higher and needs to be more precisely targeted towards the intended response range. The most preferred pKa of TEE's for oligonucleotides is 4.5 to 5.5. Still preferred are pK values between 4 and 6.5. As described above, the governing pKa for the shift in hydrophilictiy can be the pKa of a weak acidic group, namely carboxylic acids in the present invention. In cases where a shift in hyrophilicity is caused by zwitterion formation, the governing pKa is the pKa of a weak base such as pyridin, imidazol, morpholine or piperazine.

TEE's may contain one or more hydrophilic elements and the relative and absolute positioning of the hydrophilic elements may vary. In some cases, neighbouring effects may occur. Effects within the hydrophilic groups include, amongst others, pK shifts and zwitterion formation. Effects between hydrophilic groups may also include shifts in the respective pKa values. This is known to the skilled artisan and frequently observed between carboxylic acids in close proximity, e.g. when the spacing between groups is between 2 to 5 carbon atoms.

Some examples for more complex hydrophilic elements are shown below (table 4).

Table 4: Compounds 15 and 16

| | |
|---|---|
| (15) 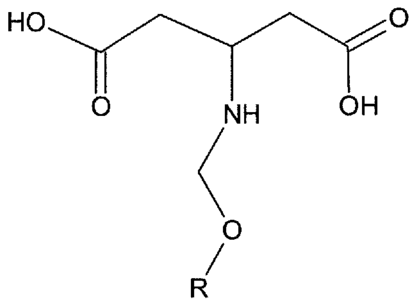 | Compound (15) β-Glutamic acid derivatives. R represents the hydrophobic element of the invention. The ether bond between R and the hydrophilic element is optional and lowers the pka of the amino group. |
| (16) 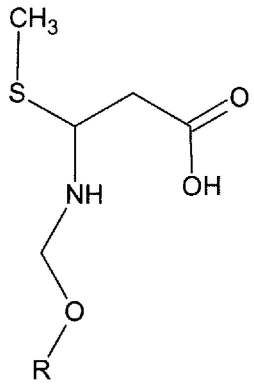 | Compound (16) 3-Amino-3-(methylthio)propanoic acid derivatives. R represents the hydrophobic element of the invention. Again, the ether bond between R and the amino group is optional but shifts the pka downwards and the same holds true for the thioether. The hydrophobic element may also be bound other positions including the methyl group at the thioether. |

TEE's with more than one hydrophilic element have larger amplitudes of hydrophilicity between their neutral and slightly acidic state. Of course, mixtures of hydrophilic elements can be combined with a single hydrophobic element. Such mixture may allow more precise adjustments in the amplitude and pH-sensitivity of the hydrophobic shift. However, too large of a number of hydrophilic elements increases the hydrophilicity of the TEE to values that can no longer be compensated with the hydrophobic shift.

Therefore, besides the amplitude of hydrophilicity between different pH values, the absolute hydrophilicity of the TEE at a first pH represents a very important aspect of the invention.

In terms of absolute hydrophilicity, the log D of the TEE itself may be varied between slightly hydrophilic at conditions of neutral or physiological pH and somewhat hydrophobic. In other words the TEE's have a logD at pH7.4 between -2 and 10.

In some aspects, the logD (7.4) is greater than 1 and in some aspects the logD(7.4) greater than 3. In other aspects of the invention, the logD(7.4) of the TEE is smaller than 10, in some aspects the logD(7.4) is smaller than 7.

In another aspect of the invention the logD of the TEE correlates with the logP or logD of the substance to be transported. In one variant of such aspect, the logD(7.4) of the TEE compensates logP or logD of the drug to be transported to an extent that the sum of both is bigger than -10. In preferred aspects, the sum is bigger than -5 and in even more preferred aspects the sum is bigger than -3. This combination alone may render the drug more amphipathic, thus improving its availability of transfection of cells. The additional contribution from the pH-sensitive element of the TEE will then facilitate a pH dependent transfection, e.g. from the endosomal pathway or at bodily sites of low pH such as in cancers or at sites of inflammation.

One or more TEE's may facilitate the transfection of a polymer, e.g. an oligonucleotide, polynucleotide, peptide or protein. The teachings from the previous paragraph can also be applied in such a case and the repetitive elements of the polymers (e.g. one or few amino acids or one or a few nucleotides) can be considered the drugs to be transported. As such, the TEE's chosen for such applications may have logD values close to the logP or log D constants of the repetitive element in question.

Alternatively, averaging effects for polymers may allow selecting a smaller number of TEE's with higher logD.

Independent from its absolute logD at pH7.4, the logD of the TEE at pH4.0 needs to exceed 0.

TEE's with a negative logD(7.4) require high amplitudes of the hydrophobic shift and such high amplitudes can be provided hydrophilic elements comprising one or more carboxyl groups.

Specific examples of preferred TEE's:

The following chemical representations of TEE's should further illustrate the teachings of the invention. However, the scope of the invention is by no means limited to the specific examples given below. Preferred TEE's have the following

attributes:

| | |
|---|---|
| Number of units in the hydrophobic element | : 4...40 |
| logD(7.4) | : -2...10 |
| (correlates to logP of the drug to be transported) | |
| logD(7,4)-logD(4) | : >1 |
| pKa for weak acids | : 2 ... 6 |
| for weak acids being used in the oligomeric TEE's in oligonucleotides | : 4 ... 6.5 |
| for weak bases with ability for zwitterion formations | : 4.5 ... 7 |
| TEE's based on carboxylic acids | |

In the invention, the TEE comprises one or more carboxylic acid groups as the hydrophilic element.

In some embodiments of such aspect, the hydrophobic element comprises a straight chain of carbon atoms. In some representations, such chain is a straight alkyl chain.

Table 5 below is analyzing logD at pH4 and pH7.4 for different chain length of the carboxylic acids.

Table 5:

| # of C | pH 4,0 | pH 7,4 | Δ |
|---|---|---|---|
| 4 | 0,71 | -1,83 | -2,54 |
| 6 | 1,77 | -0,75 | -2,52 |
| 8 | 2,84 | 0,31 | -2,53 |
| 10 | 3,9 | 1,38 | -2,52 |
| 12 | 4,96 | 2,44 | -2,52 |
| 14 | 6,02 | 3,5 | -2,52 |
| 16 | 7,09 | 4,56 | -2,53 |
| 18 | 8,15 | 5,62 | -2,53 |
| 20 | 9,21 | 6,69 | -2,52 |

It becomes apparent that chain elongation by an methylene group increases the logD by about 0.5 units. Carboxylic acids with 6 to 26 C atoms represent preferred TEE's according to the selection criteria given above. Position effects of the carboxylic acid group are less important and the carboxylic group is not mandatory the terminal group of the hydrophobic element.

In some aspects, one or more positions of the main chain of the hydrophobic element can be substituted (R-) and the impact of some substitutions is analyzed below for hexadecanoic acid (palmitic acid) derivatives (table 6).

Table 6:

| methyl side chain | | | | ethyl side chain | | | | propyl side chain | | | |
|---|---|---|---|---|---|---|---|---|---|---|---|
| # subs | pH 4,0 | pH7,4 | | # subs | pH 4,0 | pH7,4 | | # subs | pH 4,0 | pH 7,4 | |
| 0 | 7,09 | 4,56 | -2,53 | 0 | 7,09 | 4,56 | -2,53 | 0 | 7,09 | 4,56 | -2,53 |
| 1 | 7,43 | 4,91 | -2,52 | 1 | 7,96 | 5,44 | -2,52 | 1 | 8,5 | 5,98 | -2,52 |
| 2 | 7,78 | 5,26 | -2,52 | 2 | 8,84 | 6,32 | -2,52 | 2 | 9,91 | 7,38 | -2,53 |
| 3 | 8,13 | 5,6 | -2,53 | 3 | 9,72 | 7,2 | -2,52 | 3 | 11,32 | 8,79 | -2,53 |

If R= methyl, each R results in a gain in logD of about 0.35 units. If R= ethyl, such gain is about 0.88 and for R=propyl the gain is about 1.41 per substitution. It becomes apparent that addition of methylene groups in side chain also increase logD by about 0.5 units as it is the case in the main chain.

In other aspects, R comprises heteroatoms, in particular oxygen atoms and the impact for some substitutions is analyzed below for hexadecanoic acid (palmitic acid) derivatives (table 7).

Table 7:

| methoxy side chain | | | | ethoxy side chain | | | | MOE side chain | | | |
|---|---|---|---|---|---|---|---|---|---|---|---|
| # subs | pH 4,0 | pH 7,4 | | # subs | pH 4,0 | pH7,4 | | # subs | pH 4,0 | pH7,4 | |
| 0 | 7,09 | 4,56 | -2,53 | 0 | 7,09 | 4,56 | -2,53 | 0 | 7,09 | 4,56 | -2,53 |
| 1 | 6,03 | 3,35 | -2,68 | 1 | 6,56 | 3,9 | -2,66 | 1 | 6,89 | 4,31 | -2,58 |
| 2 | 4,48 | 1,8 | -2,68 | 2 | 5,54 | 2,87 | -2,67 | 2 | 6,4 | 3,81 | -2,59 |
| 3 | 2,92 | 0,24 | -2,68 | 3 | 4,52 | 1,85 | -2,67 | 3 | 5,91 | 3,32 | -2,59 |

For R= methoxy each R results in a decrease of logD of about -1.4 units. If R= ethoxy, the extra methylene group contributes about 0,5 units of logD and the resulting effect is about -0,9 units of logD. If R= methoxyethyl, the average impact per substitution is about -0,4 units of logD.

Other substitutions on the side chain may further change the logD of the chain with different impact and some examples for R are given in table 8 below (analysis based on hexadecanoic acid).

Table 8:

| R= | D (log(D)) | R= | D (log(D)) |
|---|---|---|---|
| Vinyl | +0.4 | Methoxymethyl- | -1 |
| Chloride | -0.2.... -0.5 | Ethoxymethyl- | -0.5 |
| Fluoride | -0.9 | Ethoxyethyl- | 0 |
| Bromide | -0.35.... +0.2 | Keto- | -2.2 .... -1.7 |
| Hydroxyl | -1.2 ... 2.2 | | |

The substituents itself are not pH-responsive and therefore do not contribute to the pH-response of the TEE. Also, the impact of R is independent of the pH. However, as pointed out before R may influence the pka of the hydrophilic element, thereby changing the amplitude of log(D) between physiological pH and pH4...5.

In still other aspects, heteroatoms may be part of the main chain of the hydrophobic element. In other aspects, the main chain may comprise non-saturated bonds. In still other aspects, the main chain may comprise heteroatoms in combination with subsitutions in the side chain. Such changes may influence the logD of the TEE and some variations for the main chain have been analyzed for hexadecanoic acid (table 9).

Table 9:

| double bonds (-2H) | | | | ether in main chain (PEG) | | | | ether in main chain (PPG) | | | |
|---|---|---|---|---|---|---|---|---|---|---|---|
| # subs | pH 4,0 | pH7,4 | | # subs | pH 4,0 | pH7,4 | | # subs | pH 4,0 | pH7,4 | |
| 0 | 7,09 | 4,56 | -2,53 | 0 | 7,09 | 4,56 | -2,53 | 0 | 7,09 | 4,56 | -2,53 |
| 1 | 7,11 | 4,59 | -2,52 | 1 | 5,16 | 2,54 | -2,62 | 1 | 5,51 | 2,88 | -2,63 |
| 2 | 6,58 | 3,99 | -2,59 | 2 | 3,21 | 0,57 | -2,64 | 2 | 3,9 | 1,26 | -2,64 |
| 3 | 5,95 | 3.09 | -2,86 | 3 | -0,69 | -3,33 | -2,64 | 3 | 2,3 | -0,34 | -2,64 |

A double bond therefore reduces logD by about -0.4 to 0.5 units, ether bonds in the main chain as found in repetitive strucutres derived from poly-ethylenglycol reduce logD by about 2.5 units, said effect being reduced to about 1.6 units by addition of an additional methyl group next to the ether bond, such as in the repetitive structures from polypropylenglycol.

Other substitutions on the main chain may further change the logD of the chain with different impact and some examples are given in table 10 below (analysis based on hexadecanoic acid).

Table 10:

| Element | D (log(D)) | element | D (log(D)) |
|---|---|---|---|
| -CH2- | Reference | -S- | -1,5 |
| -NH- (Aza) | -6 .... -9 | -S-S- | -1.1 |

(continued)

| Element | D (log(D)) | element | D (log(D)) |
|---|---|---|---|
| -NH-CO- (Amide) | -7.5 ... -11 | | |
| -O-CO- (Ester) | -2,7 | | |

The substituents itself are not pH-responsive and therefore do not contribute to the pH-response of the TEE. Also, the impact of R is independent of the pH. However, as pointed out before R may influence the pka of the hydrophilic element, thereby changing the amplitude of log(D) between physiological pH and pH4...5 .

The examples and analysis presented above give guidance for practicing the invention, in particular to identify structural elements as TEE's. Isoforms and position isomers are within the disclosure of the current invention. As mentioned earlier in this disclosure, neighbouring effects between substituents may occur. However, these effects are known to the skilled artisan and are described in the standard chemical literature,in databases or are part of chamical software such as ACD/Labs and others.

Ring systems

In some aspects, the hydrophobic element may form a cyclic structure such as cycloalkanes, cycloalkenes or cycloalkines or aromatic ring structures. A few representations of cyclic elements have been analyzed in the table below and more cyclic elements can be developed from known structural contributions of other elements. It is of course possible to further substitute the cyclic elements, in particular with the groups analyzed above.

Table 11:

| | **Cyclic backbone** | | | |
|---|---|---|---|---|
| | **cpd** | **pH 4,0** | **pH7,4** | Δ |
| stearic acid | | 8,15 | 5,62 | -2,53 |
| 4-undecyl cyclohexanoic acid | | 7,54 | 5,22 | -2,32 |
| 4-undecyl cyclohexenoic acid | | 7,12 | 4,39 | -2,73 |
| p-undecyl benzoic acid | | 7,51 | 4,87 | -2,64 |

Sterols

In a specific variant of the invention, the ring systems of the hydrophobic elements may be more complex ring systems such as in sterols. Again, further substitutions may be present at the sterol backbone and the analysis shown below is detailing some aspects of logD for natural occuring derivatives of sterols, e.g. hydroxyl group substitutions at positions 3,7 and 12 of the sterane backbone.

Also, the orientation of the sterol in the TEE may be different and a presence of the carboxyl group at position 26 such as in bile acids or grafted onto position 3 such as in cholesterolhemisuccinate (CHEMS) represent some instant examples (tables 12 and 13).

Table 12: Analysis of logD for bile acid derivatives used as TEE's. For analytical purposes, the 3' hydroxyl group was assumed to be methylated to model a potential drug linkage

| **bile acids** | OH in backbone | | | |
|---|---|---|---|---|
| (3'cholesterols) | **#subs** | **pH 4,0** | **pH7,4** | Δ |
| 3' methoxy cholate | 3 | 3,18 | 0,64 | -2,54 |
| 3' methoxy deoxycholate | 2 | 5,23 | 2,69 | -2,54 |
| 3' methoxy dideoxycholate | 1 | 7,27 | 4,73 | -2,54 |

Table 13: Analysis of logD for CHEMS derivatives used for TEE's. For analytical purposes, the carboxyl group in position 26 was esterified with methanol to model a potential drug linkage. In this analysis, the 3' position is esterified with succinic acid, thereby providing an unsubstituted carboxyl group as the hydrophilic element of the TEE.

| **CHEMS derivatives** (26' cholesterols) | **OH in backbone** **# subs** | **pH 4,0** | **pH7,4** | Δ |
|---|---|---|---|---|
| 26' methyl cholate | 3 | 3,65 | 1,01 | -2,64 |
| 26' methyl deoxycholate | 2 | 5,69 | 3,06 | -2,63 |
| 26' methyl dideoxycholate | 1 | 7,73 | 5,1 | -2,63 |

The position of the hydrophilic element within the TEE structure may vary. In some aspects, the hydrophilic element is located distal from the link between molecule and TEE. In other aspects, the hydrophilic element is located central within the TEE.

Use of TEE's

The TEE's can directly be linked to active pharmaceutical ingredients such as small molecules, but also to proteins, peptides, carbohydrates or nucleic acids. The conjugation with the substance to be transported can be achieved by means of chemical bonds, physical attraction or by other interactions.

A conjugation via chemical bonds can be achieved by any chemical reaction, which may lead to linker groups including, without limitation, -O-, -S-, - NH-, -NR-, -N(H)C(O)-, -C(O)O-, -OC(O)N(H)-, -C(O)-, -C(O)-N(H)-,-N(H)-C(O)-O-, -CH=N-, -O-C(O)-, -N=CH-, -OP(O)(OH)O-, -P(O)(OH)O-, - OP(O)(OH)-, -OS(O)(O)O-, -S(O)(O)O-, -OS(O)(O)- and/or -S-S-.

Methods of synthesize such conjugates comprising a transfection enhancer element as described above include coupling reactions that are well-known to those skilled in the art and may vary depending on the starting material and coupling component employed. Typical reactions are esterification, amidation, etherification, or reductive amination.

TEE's can also be conjugated to carrier systems sequestering the actual active ingredient. Such carrier systems may comprise polymers or copolymers or block-polymers of various chemistries. Such carriers may further comprise supramolecular aggregates of polymers, e.g. polymer based beads. In the invention, the carrier systems are as defined in the claims.

Other carrier systems may comprise biocompatible carriers suitable for the transport of small molecules, peptides, proteins or nucleic acids. Examples for such carriers include, without limitation cationic polymer based systems such as polyethylenimins of linear or branched type (e.g. Boussif et al, Proc. Natl. Acad. Sci., 92(16), 7297-7301, 1995 or Godbey et al., J. Control. Release, 60(2-3), 149-160, 1999), chitosan and derivatives thereof (e.g. Janes et al., Adv. Drug Deliv. Rev., 47(1), 83-97, 2001), cationic cyclodextrins and derivatives thereof, some of them being disclosed in Hu-Lieskovan et al., Cancer Res., 65(19), 8984-8992, 2005. Examples may further include carriers based on gelatin, collagen or atelocollagen, some of them being disclosed e.g. in Kaul et al., Pharm. Res., 22(6), 951-961, 2005) or Sano et al., Adv. Drug Deliv. Rev., 55(12), 1651-1677, 2003.

It is possible to use carriers derived from viral capsids for the delivery of polynucleotides or oligonucleotides and TEE's of this invention can be combined with viral capsids, such combination can be done before or after assembly of the capsid.

In one aspect of the invention, one or more TEE's can be grafted on the carrier systems or polymers using chemical conjugation techniques and methods for carrying out such couplings have extensively been reported in the literature, e.g. by G. T. Hermanson, Bioconjugate Techniques, Academic Press, 1996.

In yet another aspect, one or more TEE's may become an integral part of the carrier system. In one embodiment, TEE's further comprising a polycationic element are combined with polyanions to form a precipitate, a complex controlled in size or a stoichiometric associate. Examples for polycationic elements include without limitation polyamines, eg. spermine, dispermine, trispermine, tetraspermin, oligospermine, thermine, spermidine, disperminidine, trispermindine, oligospermidin, putrescine, polylysine , polyarginine, polyethylenimine of branched or linear type or polyallylamine. It is known in the art that polyamines form complexes with oligonucleotides or polynucleotides. It is also known that derivatives of spermine, spermidine or putrescine, such as for example lipopolyamines still are able to form complexes with oligonucleotides or polynucleotides. Lipopolyamines include, without limitation, cholesteryl polyamine carbamates or the commercially available lipopolyamine compounds DOSPER, DOGS or DOSPA. TEE derivatives for such use include, but are not limited to the compounds 24 - 28 (table 14).

Table 14: Compounds 24-28

| |
|---|
| 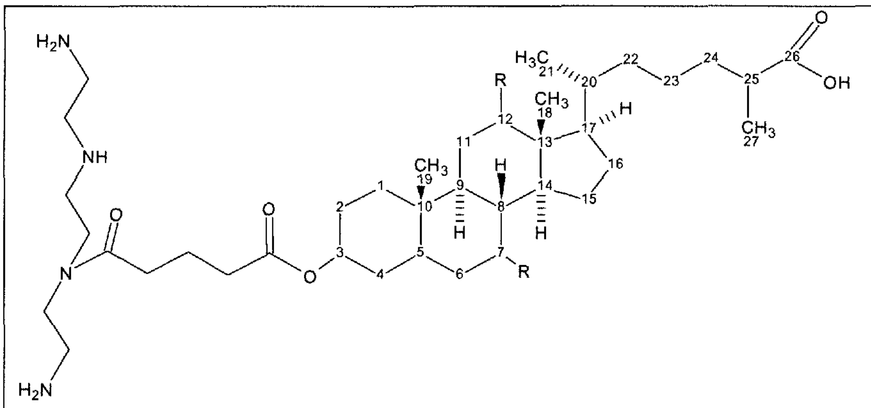<br>Compound (24), R is -H or -OH |
| 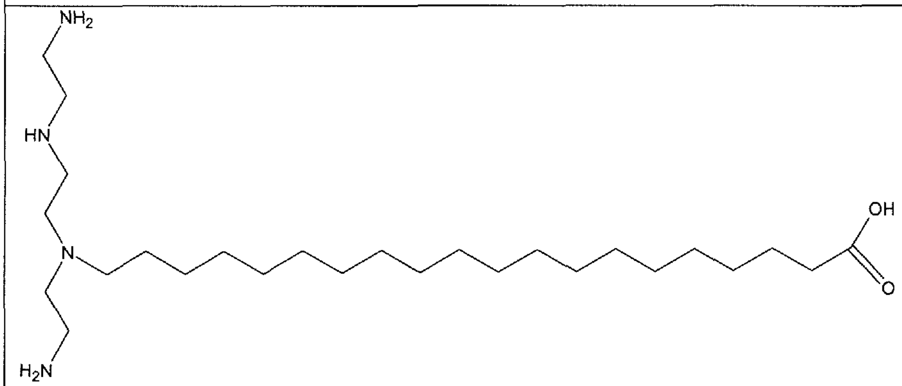<br>Compound (25) |

| |
|---|
| 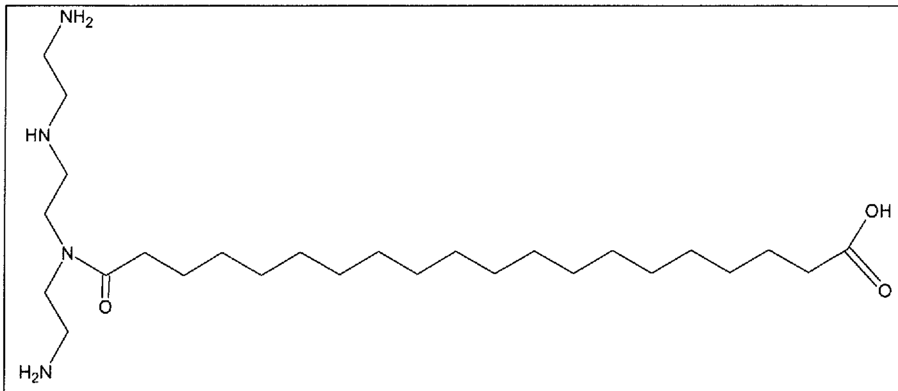 Compound (26) |
| 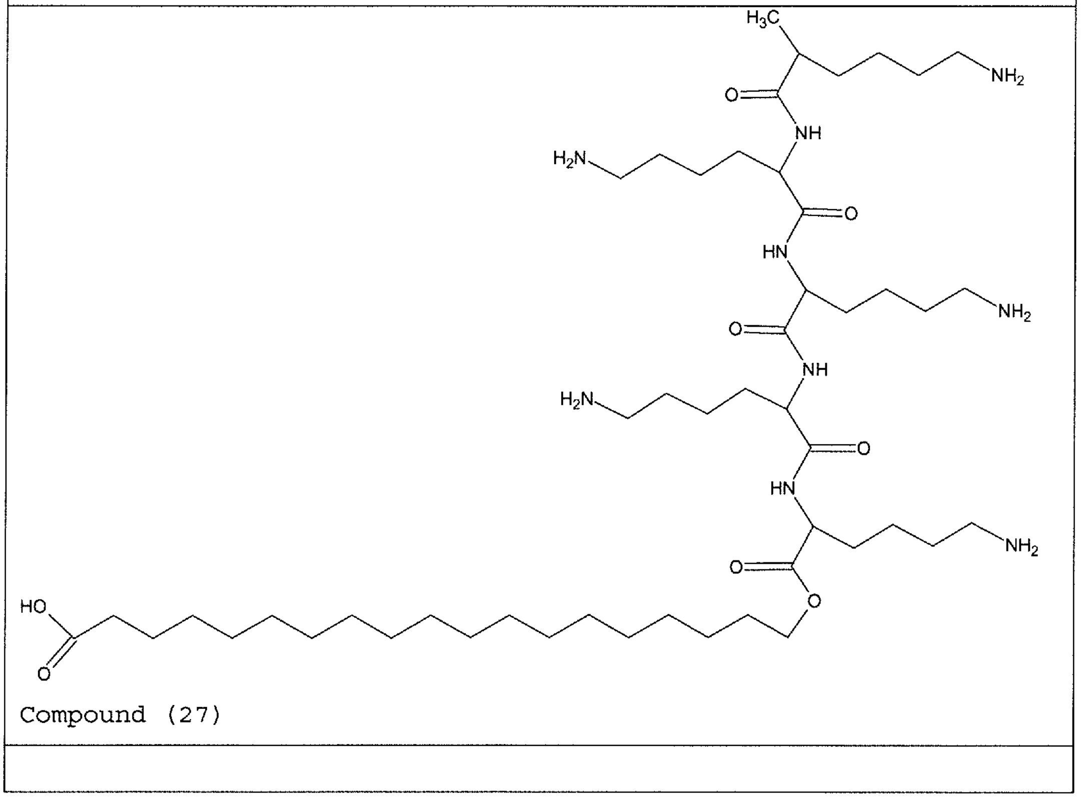 Compound (27) |
| |

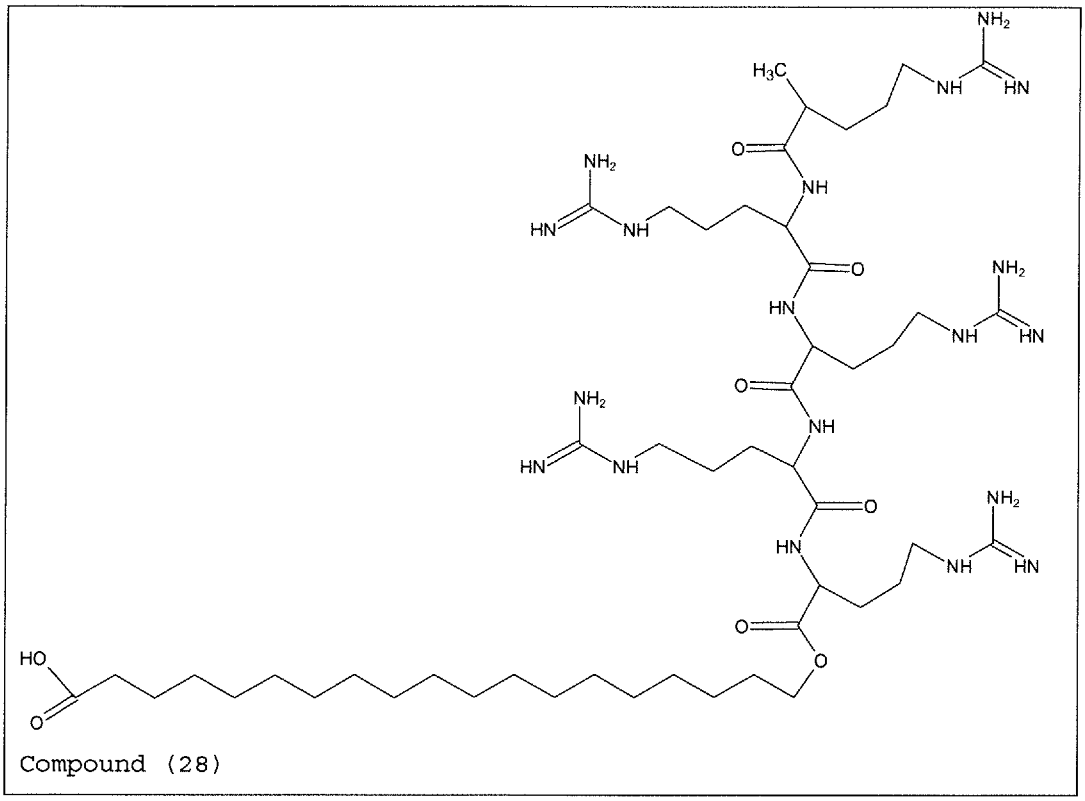

Compound (28)

In another aspect of the invention the active ingredient to be transported can be a polymer or oligomer itself, e.g. a nucleic acid, a peptide or a protein.

Nucleic Acids modified with TEE's

In one embodiment described herein, TEE's are grafted onto nucleic acids. In one aspect of such embodiment, TEE's are grafted onto the phosphate backbone of nucleic acids and modify the internucleoside linkage. A number of different variants for the internucleoside linkage are known to the skilled artisan. The binding and non-binding oxygens can be exchanged against sulphur or nitrogen atoms. It is further possible directly couple the TEE to the phosphorus atom as in phosphonoalkyl compounds. An internucleoside linkage further comprising a TEE can therefore be written as:

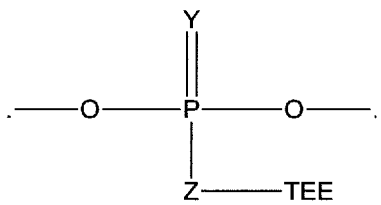

wherein Y is O or S; Z is absent or selected from -O-, -S-, -NH-, NR21R22, -C(O)-, -N(H)C(O)-, -C(O)N(H)-, -C(O)O-, -OC(O)N(H)-, -C(O)N(H)-, - N(H)C(O)O-, -CH=N-, -O-C(O)-, -N=CH- or -S-S-, wherein R21 or R22 are independently absent, H or C1-C6 alkyl and the TEE is defined as above in (VII) or (VIII).

In one variant, TEE's for such use comprise straight alkyl chains of 6 to 16 C-atoms and further comprise a single carboxyl group. In one specific embodiment, the carboxyl group is situated at the terminal end of the alkyl chain and Z is oxygen, as in compound (29). The methyl group in (29) represents the 5' methylene group of the next nucleotide.

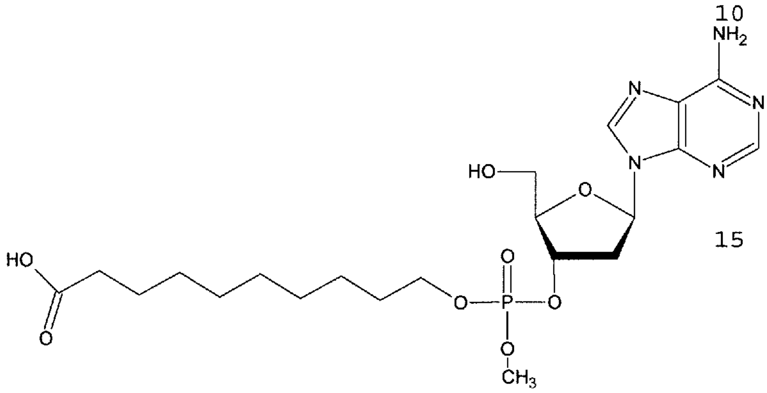

Compound (29):
C10-carboxyl-deoxyadenin.
The TEE is decanoic acid.

The 2'positions of the nucleosides represent alternative grafting positions for a TEE and such structures can be written as :

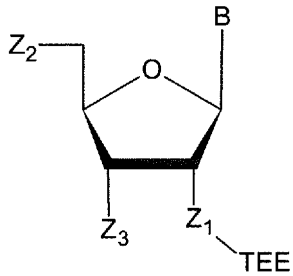

wherein B is a nucleobase such as adenin, guanin, cytosin, thymine or uracil; Z2 and Z3 are internucleoside linkages selected from phosphodiesters, phosphorothioates, phosphorodithioates or a phosphoamidates and Z1 is absent or selected from -O-, -S-, -NH-, NR23R24, -C(O)-, -N(H)C(O)-, -C(O)N(H)-, -C(O)O-, -OC(O)N(H)-, -C(O)N(H)-,-N(H)C(O)O-, -CH=N-, -O-C(O)-, -N=CH- or -S-S-, wherein R23 or R24 are independently absent, H or C1-C6 alkyl and the TEE is defined as in (VII) or (VIII).

In some embodiments the TEE moieties may be attached to the nucleotides via linking groups. Nucleotides in combination with long chain carboxylic acids are known in the state of the art, but were used for conjugating nucleic acids with other substances, e.g. for targeting of a cellular receptor. Consequently, only a limited number of such TEE modified nucleotides were incorporated into oligonucleotides or polynucleotides. For example oligonucleotides having pre-activated carbonyl linkers are disclosed in US 6320041. Oligonucleotides having 5'carboxyl linker of 10 C atoms are commercially available at TriLink Biotechnologies, San Diego, USA.

In other aspects of the invention, TEE's of a structural origin different of (VII) and (VIII) can be used to enhance the transfection of nucleic acids.

In other aspects of this disclosure, nucleobases are absent and TEE's are grafted directly onto a phosphoribose or phosphodexyribose backbone, replacing the former nucleobases at C1. Examples for such structures include, without limitation the following chemical representations (Compound (30), wherein the formula represent a portion of a larger oligonucleotide:

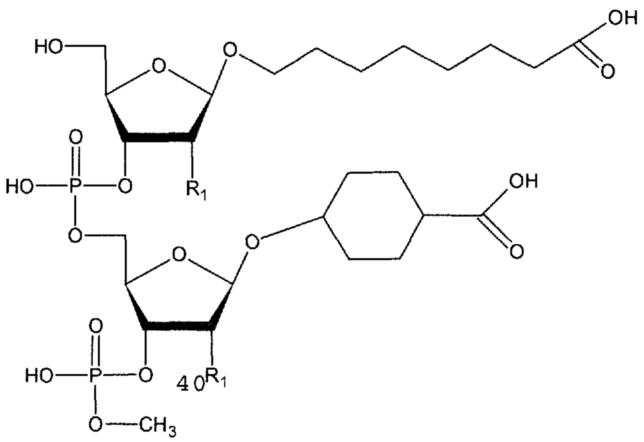

Compound (30)

Further described herein is the use of one or multiple TEE's grafted onto a single oligonucleotide or polynucleotide for enhancing the transfection of such substances. Further described are conjugates of oligonucleotides or polynucleotides with one or more TEE's selected from the different chemical representations given throughout this disclosure. In some aspects conjugates of oligonucleotides with one TEE may comprise a TEE other than a saturated, straight chain 1-carboxylic acid. In other aspects of the invention the use of transfection enhancer elements comprising saturated, straight chain 1-carboxylic acid for the in vivo, in vitro or ex vivo transfection of oligonucleotides may be preferred.

In preferred embodiments, 2 or more TEE's with the chemical representations given throughout this description are conjugated to the oligonucleotide. In some aspects of such embodiments, the TEE's are alkylcarboxylic acids with 6 or more carbon atoms, in more preferred embodiments of such aspect, the TEE's comprise 8 to 16 carbon atoms. In specific aspects of the invention the TEE's are alkylcarboxylic acids with 4 or more carbon atoms. In other aspects of such embodiments the TEE's may be sterol-based, including but not limited to bile acid derivatives as shown below (Compounds (31) and (32)).

In more preferred embodiments, 4 or more TEE's with the chemical representations given throughout the description are conjugated to the oligonucleotide. In some aspect of such embodiments, the TEE's are alkylcarboxylic acids with 6 or more carbon atoms, in more preferred embodiments of such aspect, the TEE's comprise 8 to 16 carbon atoms. In specific aspects of the invention the TEE's are alkylcarboxylic acids with 4 or more carbon atoms. In other aspects of such embodiments the TEE's may be sterol-based, including but not limited to bile acid derivatives as shown below (Compounds (31) and (32)).

In other preferred embodiments, 6 or more TEE's with the chemical representations given throughout the description are conjugated to the oligonucleotide. In some aspect of such embodiments, the TEE's are alkylcarboxylic acids with 6 or more carbon atoms, in more preferred embodiments of such aspect, the TEE's comprise 8 to 16 carbon atoms. In specific aspects of the invention the TEE's are alkylcarboxylic acids with 4 or more carbon atoms. In other aspects of such embodiments the TEE's may be sterol-based, including but not limited to bile acid derivatives as shown below (Compounds (31) and (32)).

In some embodiments described herein, all nucleobases in the oligonucleotide are modified with TEE's with the chemical representations given throughout the invention. In some aspect of such embodiments, the TEE's are alkylcarboxylic acids with 6 or more carbon atoms, in more preferred embodiments of such aspect, the TEE's comprise 8 to 16 carbon atoms. In specific aspects of the invention the TEE's are alkylcarboxylic acids with 4 or more carbon atoms. In other aspects of such embodiments the TEE's may be sterol-based, including but not limited to bile acid derivatives as shown below (Compounds (31) and (32)).

In some preferred embodiments, no more than 2/3 of all nucleobases in the oligonucleotide are modified with TEE's with the chemical representations given throughout the description. In some aspect of such embodiments, the TEE's are alkylcarboxylic acids with 6 or more carbon atoms, in more preferred embodiments of such aspect, the TEE's comprise 8 to 16 carbon atoms. In specific aspects of the invention the TEE's are alkylcarboxylic acids with 4 or more carbon atoms. In other aspects of such embodiments the TEE's may be sterol-based, including but not limited to bile acid derivatives as shown below (Compounds (31) and (32)).

In some other preferred embodiments, no more than 1/3 of all nucleobases in the oligonucleotide are modified with TEE's with the chemical representations given throughout the description. In some aspect of such embodiments, the TEE's are alkylcarboxylic acids with 6 or more carbon atoms, in more preferred embodiments of such aspect, the TEE's comprise 8 to 16 carbon atoms. In specific aspects of the invention the TEE's are alkylcarboxylic acids with 4 or more carbon atoms.In other aspects of such embodiments the TEE's may be sterol-based, including but not limited to bile acid derivatives as shown below (Compounds (31) and (32)).

In yet other embodiments described herein, only nucleobases at the flanks of the oligonucleotides or polynucleotides are modified with TEE's leading to gapmer structures. In a preferred aspect of this embodiment no more than 2/3 of all nucleobases were modified with TEE's with the chemical representations given throughout the description. In some aspect of such embodiments, the TEE's are alkylcarboxylic acids with 6 or more carbon atoms, in more preferred embodiments of such aspect, the TEE's comprise 8 to 16 carbon atoms. In specific aspects of the invention the TEE's are alkylcarboxylic acids with 4 or more carbon atoms.

In other aspects of such embodiments the TEE's may be sterol-based, including but not limited to bile acid derivatives as shown below (Compounds (31) and (32)).

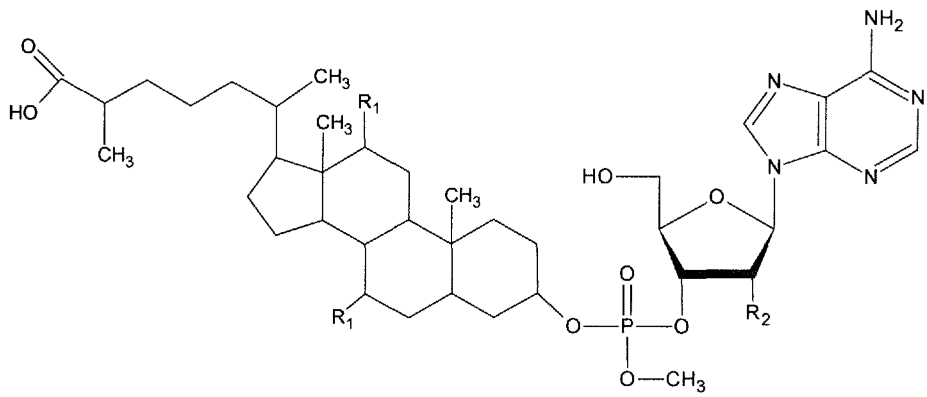

Compound (31): Nucleotides modified with a bile acid or derivative thereof. R1 and R2 may independently be H, OH, methyl-, ethyl-, propyl-, isopropyl-, methoxy-, ethoxy-, methoxymethyl-, ethoxymethyl-, methoxyethyl-, ethoxyethyl- and vinyl- or halogen. In further preferred variants, R1 is -H or -OH or mixtures of both and R2 is -H, -OH, -OMe or methoxyethyl.

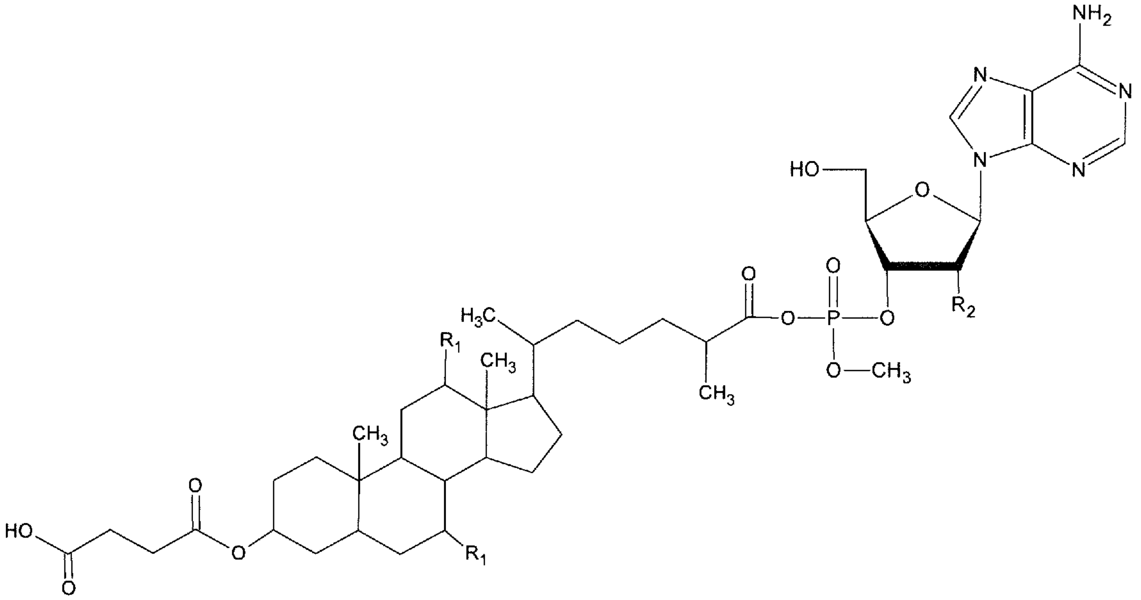

Compound (32): Nucleotides modified with a bile acid or derivative thereof. R1 and R2 may independently be H, OH, methyl-, ethyl-, propyl-, isopropyl-, methoxy-, ethoxy-, methoxymethyl-, ethoxymethyl-, methoxyethyl-, ethoxyethyl- and vinyl- or halogen. In further preferred variants, R1 is -H or -OH or mixtures of both and R2 is -H, -OH, -OMe or methoxyethyl.

All nucleobases can be used in conjugation with the TEE's and the physicochemical differences between the individual nucleobases are rather small compared to the impact of the modifying TEE's. The logD values for structural elements of an oligonucleotide can be predicted using known values from the literature and standard algorithms. The carboxylmethyl derivative of a nucleotide has comparable values of logD(7.4) of the unmodified phosphate variant (~ -5) and every methylene group added to the hydrophobic chain of the TEE raises the logD about 0.5 units. Figure 5 gives a more detailed analysis for the different nucleobases. It becomes apparent from the figure that the carboxyl groups from the TEE increase the logD with about 2 to 2.5 units when exposed to the slightly acidic pH.

Figure 6 gives a more detailed analysis for a short oligonucleotide backbone fragment being fully modified with C8 carboxyl TEE's. The structures for the analysis are derived from compound 33.

Compound (33):

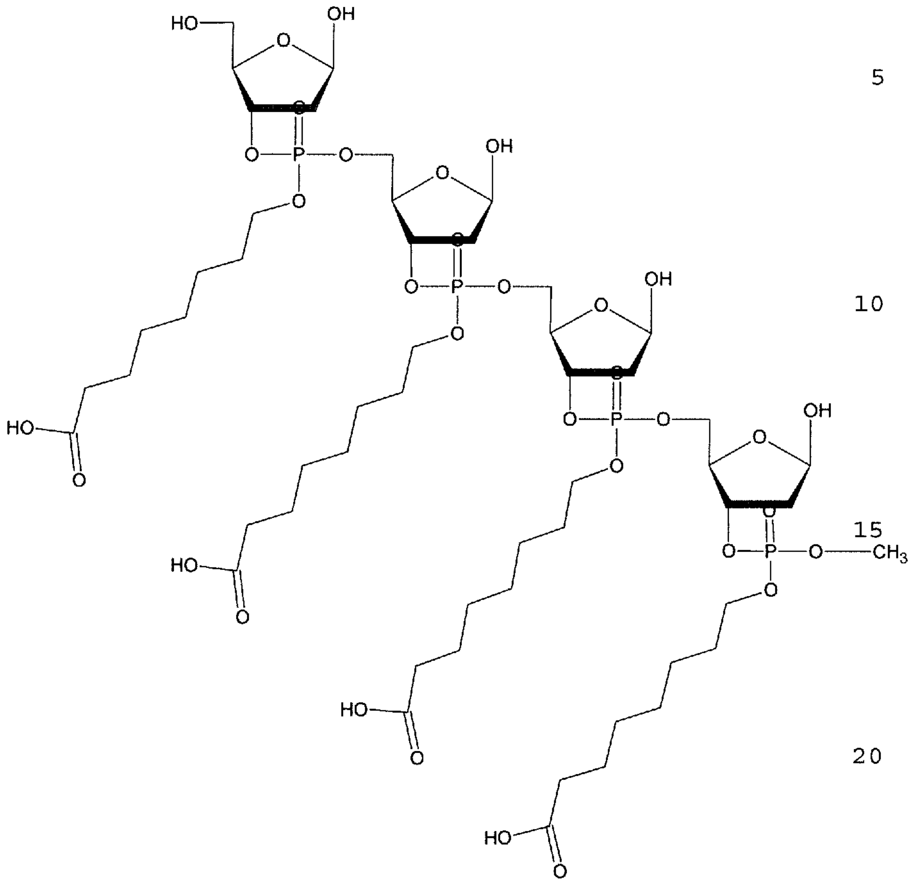

In contrast to the unmodified structures, the TEE modified backbone is already less hydrophobic at neutral pH, but still very hydrophilic. More importantly and in contrast to the unmodified form, the TEE modified structure is now sensitive to a shift in pH between 4 and 7. Under slightly acidic conditions the structure has very limited hydrophilicity and is now able to diffuse through biological membrane.
Moreover, any extension of the fragment towards longer chains, although increasing the hydrophilicity at pH 7, does increase the hydrophobicity at pH 4 to a very limited extent.

The following analysis is further illustrating the design of oligonucleotides comprising TEE's s grafted to their internucleoside linkages. Most of the hydrophilicity of these substances is contributed from the phosphate/nucleoside backbone. In an abasic DNA backbone each monomer contributes about logD of -2.5. The four nucleobases have an average contribution of -1.3 (pH7.4) or -1.7 (pH4), thus the logD value per nucleotide is -3.8 (pH7.4) or -4.2 (pH4). An oligonucleotide therefore becomes more polar when exposed to low pH conditions. For a 20mer oligodeoxyribonucleotide the total polarity is about (log D) -79 (pH7.4) or -87 (pH4). A first reduction in polarity can be achieved by incorporation of sulphur atoms into the internucleoside linkage. A 20mer phosphorothioate (PTO) modified oligodeoxyribonucleotide has a log D of -67 (pH7.4) or -76 (pH4).

TEE's for the enhancement of membrane permeability of an oligonucleotide are selected to minimize the overall polarity of the molecule and to minimize or revert the increase in polarity that occurs when the oligonucleotide is exposed to low pH. LogD values for a series of related TEE's s grafted to the internucleoside linkage are reported in the table below. The analysis was done for 20mers of deoxyribonucleotides, wherein the degree of modification was kept at 50%. The total backbone was assumed to be of phosphodiester type for the analysis of phospho- or phosphono-TEE linkages. In contrast, the backbone was assumed to be of PTO type for the analysis of thiophospho- or thiophosphono-TEE linkages. In detail, the following linkages were analyzed:

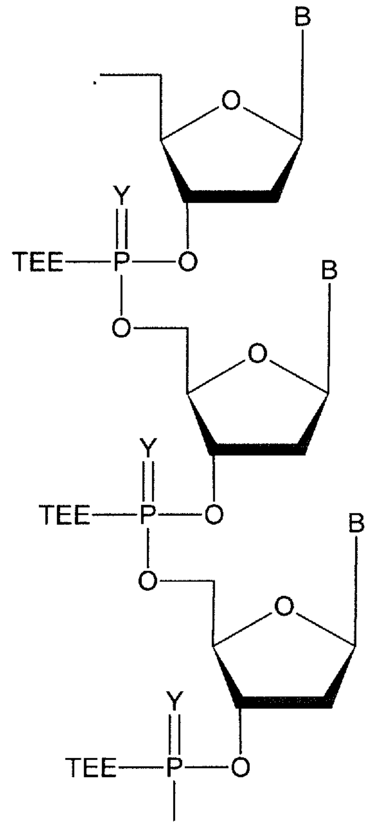

Wherein the formula represents a portion of a longer oligonucleotide and B is any nucleobase selected from adenine, guanine, thymine, cytosine or uracile; Y is O for phosho- or phosphono compounds or S for thiosphopho- or thiophosphono compounds and position of the TEE can be formic acid, acetic acid, butyric acid, hexanoic acid, cyclohexanoic acid, octanoic acid, 1-methyl-4-acetyl-cyclohexanoic acid, decanoic acid, dodecanoic acid, myristic acid or palmic acid , said structural fragment comprising 1,2,4,6,7,8,9,10,12,14 or 16 carbon atoms, respectively.

| # of C in TEE | phosphono | | phospho | | thiophosphono | | thiophospho | |
|---|---|---|---|---|---|---|---|---|
| pH | 4 | 7,4 | 4 | 7,4 | 4 | 7,4 | 4 | 7,4 |
| 1 | -91 | -83 | -86 | -78 | -85 | -77 | -80 | -72 |
| 2 | -86 | -81 | -90 | -78 | -67 | -62 | -83 | -71 |
| 4 | -74 | -77 | -72 | -74 | -56 | -58 | -65 | -67 |
| 6 | -71 | -74 | -66 | -69 | -53 | -55 | -59 | -62 |
| 7 | -67 | -69 | | | -48 | -51 | | |
| 8 | -64 | -66 | -56 | -58 | -45 | -48 | -49 | -51 |
| 9 | -60 | -62 | | | -41 | -44 | | |
| 10 | | | -45 | -47 | | | -38 | -41 |
| 12 | -35 | -37 | -35 | -37 | -24 | -26 | -27 | -30 |
| 16 | | | -13 | -16 | | | | |
| *no TEE* | *-86,8* | -79,2 | *-86,8* | -79,2 | *-76,5* | *-67,3* | -76,5 | *-67,3* |

| gain from TEE | phosphono | | phospho | | thiophosphono | | thiophospho | |
|---|---|---|---|---|---|---|---|---|
| pH | 4 | 7,4 | 4 | 7,4 | 4 | 7,4 | 4 | 7,4 |
| 1 | -4 | -4 | 1 | 1 | -8 | -10 | -4 | -5 |
| 2 | 1 | -2 | -3 | 1 | 9 | 5 | -6 | -4 |
| 4 | 12 | 2 | 15 | 5 | 21 | 9 | 12 | 0 |

(continued)

| gain from TEE | phosphono | | phospho | | thiophosphono | | thiophospho | |
|---|---|---|---|---|---|---|---|---|
| pH | 4 | 7,4 | 4 | 7,4 | 4 | 7,4 | 4 | 7,4 |
| 6 | 15 | 5 | 20 | 10 | 24 | 12 | 17 | 6 |
| 7 | 20 | 10 | | | 28 | 17 | | |
| 8 | 23 | 13 | 31 | 21 | 31 | 20 | 28 | 16 |
| 9 | 27 | 17 | | | 36 | 24 | | |
| 10 | | | 42 | 33 | | | 38 | 27 |
| 12 | 52 | 42 | 52 | 42 | 53 | 41 | 49 | 37 |
| 16 | | | 73 | 63 | | | | |

Alkylcarboxylic acids comprising only one or two carbon atoms do not improve the logD of the 20mer and it becomes apparent from the data that TEE's s comprising higher alkyls are needed to substantially reduce the polarity of the oligodeoxyribonucleotide.

Currently developed oligonucleotides are often modified in their 2'position as 2' MOE, 2' F or 2' 4' methoxyl bridged compounds (LNA) and shorter oligonucleotides down to 16 or 12mers have been shown to be taken up in vivo. These observations support a logD of about -60 that is needed to afford cell permeability in vivo. It becomes clear from the analysis above which TEE/internucleoside linkage combinations should be selected to achieve a substantial improvement in the membrane permeability of an oligonucleotide. TEE's for phosphono linkages require 9 or more carbon atoms, TEE's for phospho linkages work with 7 or more carbon atoms. TEE' s for thiophosphono linkages require 4 or more carbon atoms to avoid a polarity increase at low pH conditions. TEE's grafted onto a thiophospho linkage need 6 or more carbon atoms in an alkyl group.

It also become apparent from the analysis that there is no need for a specific configuration of the alkyl group, since very similar properties towards a logD can be observed for cycloalkanes and linear alkanes.Similar results are obtained for the branched isoalkyl residues.

The table below presents more detailed data for the individual configurations of TEE's grafted onto the internucleoside linkages described above. LogD values have been calculated for pH 7.4 and pH 4 for abasic nucleoside oligomers comprising 1 to 4 elements. From these initial data, the contribution of a monomers (impact) as well as the virtual starting logD at chain length zero (offset) was calculated. The difference between the impact factors at pH4 and 7.4 reflects the pH sensitive effect of the hydrophilic element of the TEE. Positive values of about 1 indicate functional TEE's that have the expected hydrophilic-hydrophobic shift mentioned in the description of this invention.

The chemical names and structures have been described above. Further abbreviations in the table are: 2'OMe: 2' O-methyl; 2' MOE: 2'0-methoxyethyl. As above, homogenous modifications with respect to thiolation of the internucleoside linkages were used.

| | | abasic | | | | | | |
|---|---|---|---|---|---|---|---|---|
| | | uniform 2' modified | | | | | | |
| lenght of oligonucleotide | pH | 1 | 2 | 3 | 4 | impact | offset | (impact) |
| deoxy | 7,4 | -5,6 | -8,1 | -10,6 | -13,2 | -2,5 | -3,0 | 0,0 |
| | 4 | -4,6 | -7,9 | -10,5 | -13,0 | -2,5 | -2,9 | |
| 2'OH | 7,4 | -5,8 | -8,9 | -11,9 | -15,0 | -3,0 | -2,8 | 0,0 |
| | 4 | -5,0 | -8,8 | -11,9 | -14,9 | -3,0 | -2,8 | |
| PTO/DNA | 7,4 | -5,3 | -7,2 | -9,1 | -11,1 | -1,9 | -3,3 | -0,1 |
| | 4 | -3,6 | -6,9 | -9,1 | -11,1 | -2,0 | -3,0 | |
| PTO/RNA | 7,4 | -5,2 | -7,7 | -10,1 | -12,5 | -2,4 | -2,8 | 0,0 |
| | 4 | -4,4 | -7,6 | -10,1 | -12,5 | -2,4 | -2,8 | |
| 2' Ome | 7,4 | -5,3 | -7,7 | -10,1 | -12,5 | -2,4 | -2,9 | 0,0 |
| | 4 | -4,4 | -7,6 | -10,1 | -12,5 | -2,4 | -2,9 | |
| 2' O propyl | 7,4 | -4,5 | -6,2 | -7,8 | -9,5 | -1,6 | -2,9 | 0,0 |
| | 4 | -3,7 | -6,1 | -7,8 | -9,5 | -1,6 | -2,9 | |
| 2'MOE | 7,4 | -6,0 | -9,0 | -12,1 | -15,2 | -3,1 | -2,9 | 0,0 |

(continued)

| | | abasic | | | | | | |
|---|---|---|---|---|---|---|---|---|
| | | uniform 2' modified | | | | | | |
| lenght of oligonucleotide | pH | 1 | 2 | 3 | 4 | impact | offset | (impact) |
| | 4 | -5,1 | -8,9 | -12,1 | -15,2 | -3,1 | -2,9 | |
| phosphonoformiate | 7,4<br>4 | -6,1<br>-5,5 | -9,0<br>-9,0 | -13,5<br>-13,4 | -16,4<br>-16,3 | -2,9<br>-2,9 | -4,7<br>-4,6 | 0,0 |
| phosphonoacetate | 7,4 4 | -5,7<br>-2,9 | -8,5<br>-5,3 | -11,2<br>-7,8 | -13,9<br>-10,2 | -2,7 -2,4 | -3,1 -0,5 | 0,3 |
| phosphonobutyrate | 7,4 4 | -4,5<br>-1,8 | -7,7<br>-3,1 | -10,0<br>-4,4 | -12,3<br>-5,7 | -2,3 -1,3 | -3,1 -0,5 | 1,0 |
| phosphonohexanoate | 7,4<br>4 | -4,1<br>-1,5 | -7,1<br>-2,5 | -9,1<br>-3,5 | -11,1<br>-4,5 | -2,0<br>-1,0 | -3,1<br>-0,5 | 1,0 |
| phosphonooctanoate | 7,4<br>4 | -3,2<br>-0,7 | -5,5<br>-1,0 | -6,9<br>-1,2 | -8,1<br>-1,4 | -1,2<br>-0.2 | -3,1<br>-0,5 | 1,0 |
| phosphonododecanoate | 7,4<br>4 | -0,3<br>2,2 | 0,3<br>4,9 | 1,9<br>7,6 | 3,6<br>10,2 | 1,7<br>2,7 | -3,1<br>-0,5 | 1,0 |
| phosphonocydohexanoate | 7,4<br>4 | -3,5<br>-1,0 | -6,1<br>-1,6 | -7,7<br>-2,1 | -9,3<br>-2,6 | -1,5<br>-0,5 | -3,1<br>-0,5 | 1,0 |
| phosphono (1-methyl 4 acetyl)cydohexanoate | 7,4<br>4 | -2,9<br>-0,3 | -4,7<br>-0,1 | -5,6<br>0,1 | -6,4<br>0,3 | -0,8<br>0,2 | -3,1<br>-0,5 | 1,0 |
| phospho-formiate | 7,4<br>4 | -5,6<br>-4,7 | -8,1<br>-8,0 | -10,5<br>-10,5 | -13,0<br>-13,0 | -2,5<br>-2,5 | -3,2<br>-3,1 | 0,0 |
| phosphoacetate | 7,4<br>4 | -5,5<br>-3,3 | -8,0<br>-6,3 | -10,4<br>-9,2 | -12,9<br>-12,1 | -2,4<br>-2,9 | -3,1<br>-0,6 | -0,4 |
| phospho-butyrate | 7,4<br>4 | -4,3<br>-1,6 | -7,2<br>-2,6 | -9,3<br>-3,7 | -11,3<br>-4,7 | -2,0<br>-1,1 | -3,1<br>-0,5 | 1,0 |
| phosho-hexanoate | 7,4<br>4 | -3,6<br>-1,0 | -6,6<br>-2,0 | -7,7<br>-2,0 | -9,2<br>-2,5 | -1,5<br>-0,5 | -3,1<br>-0,5 | 1,0 |
| phosphooctanoic acid | 7,4<br>4 | -2,4<br>0,1 | -3,9<br>0,6 | -4,5<br>1,2 | -4,9<br>1,7 | -0,4<br>0,6 | -3,1<br>-0,5 | 1,0 |
| phospho-decanoic acid | 7,4<br>4 | -1,4<br>1,2 | -1,8<br>2,8 | -1,3<br>4,4 | -0,6<br>6,0 | 0,7<br>1,6 | -3,4<br>-0,5 | 0,9 |
| phospho-dodecanoic acid | 7,4<br>4 | -0,3<br>2,2 | 0,3<br>4,9 | 1,9<br>7,6 | 3,6<br>10,2 | 1,7<br>2,7 | -3,1<br>-0,5 | 1,0 |
| phospho-hexadecanoic acid | 7,4<br>4 | 1,8<br>4,3 | 4,6<br>9,1 | 8,3<br>13,9 | 12,1<br>18,7 | 3,8<br>4,8 | -3,1<br>-0,5 | 1,0 |
| Thiophosphonoformiate | 7,4<br>4 | -5,5<br>-4,9 | -7,8<br>-7,8 | -11,7<br>-11,5 | -14,0<br>-13,9 | -2,3<br>-2,3 | -4,7<br>-4,6 | 0,0 |
| Thiophosphonoaceate | 7,4<br>4 | -3,8<br>-1,0 | -4,8<br>-1,6 | -5,6<br>-2,2 | -6,5<br>-2,8 | -0,8<br>-0,6 | -3,1<br>-0,5 | 0,3 |
| Thiophosphonobutyrat | 7,4<br>4 | -2,6<br>0,1 | -4,0<br>0,6 | -4,5<br>1,1 | -4,9<br>1,7 | -0,4<br>0,6 | -3,1<br>-0,5 | 1,0 |

(continued)

| | | abasic | | | | | | |
|---|---|---|---|---|---|---|---|---|
| | | uniform 2' modified | | | | | | |
| lenght of oligonucleotide | pH | 1 | 2 | 3 | 4 | impact | offset | (impact) |
| Thiophosphonohexanoate | 7,4 | -2,2 | -3,4 | -3,6 | -3,7 | -0,2 | -3,1 | 1,0 |
| | 4 | 0,4 | 1,2 | 2,1 | 2,9 | 0,9 | -0,5 | |
| Thiophosphonocyclohexanoate | 7,4 | -1,7 | -2,4 | -2,2 | -1,9 | 0,3 | -3,1 | 1,0 |
| | 4 | 0,9 | 2,2 | 3,5 | 4,8 | 1,3 | -0,5 | |
| Thiophosphono(1 methyl 4 acetyl)cyclohexano | 7,4 | -1,0 | -1,0 | 0,0 | 1,0 | 1,0 | -3,1 | 1,0 |
| | 4 | 1,6 | 3,6 | 5,6 | 7,7 | 2,0 | -0,5 | |

Starting from this set of data, higher oligomers were analyzed that also incorporated average nucleobases. For the latter, the logD coefficients reported above (-1.3 at pH7.4 and -1.7 at pH4) were used. The table below reports data for 8mers to 20mers that comprise homogenously modified internucleoside linkages and for 20mers with about 50% degree of modification. The specific appearance in the sequence of internucleoside linkages is of no substantial relevance to the logD values reported here, the modified linkages might be dispersed statistically throughout the oligomer, they might concentrate on one end, or gapmers with modified flanks and unmodified central regions may be formed.

| | | **average nucleobase use** | | | | | |
|---|---|---|---|---|---|---|---|
| | | **uniform 2' modified** | | | | | **50% mod.** |
| lenght of oligonucleotide | pH | 8 | 10 | 12 | 15 | 20 | 20 |
| deoxy | 7,4 | -33,5 | -41,1 | -48,7 | -60,2 | -79,2 | -79,2 |
| | 4 | -36,4 | -44,8 | -53,2 | -65,8 | -86,8 | -86,8 |
| 2'OH | 7,4 | -37,3 | -45,9 | -54,5 | -67,4 | -89,0 | -84,2 |
| | 4 | -40,3 | -49,7 | -59,1 | -73,2 | -96,7 | -91,8 |
| PTO / DNA | 7,4 | -28,9 | -35,3 | -41,7 | -51,3 | -67,3 | -73,1 |
| | 4 | -32,4 | -39,8 | -47,1 | -58,1 | -76,5 | -81,6 |
| PTO/RNA | 7,4 | -32,5 | -39,9 | -47,3 | -58,4 | -77,0 | -78,2 |
| | 4 | -35,5 | -43,7 | -51,9 | -64,2 | -84,7 | -85,8 |
| 2' Ome | 7,4 | -32,3 | -39,7 | -47,1 | -58,1 | -76,5 | -77,9 |
| | 4 | -35.4 | -43,5 | -51,7 | -63,9 | -84,2 | -85,5 |
| 2' O propyl | 7,4 | -26,2 | -32,0 | -37,8 | -46,6 | -61,1 | -70,2 |
| | 4 | -29,3 | -35,9 | -42,4 | -52,3 | -68,8 | -77,8 |
| 2'MOE | 7,4 | -37,6 | -46,3 | -55,0 | -68,0 | -89,7 | -84,5 |
| | 4 | -40,7 | -50,1 | -59,6 | -73,7 | -97,4 | -92,1 |
| | | | | | | | |
| phosphonoformiate | 7,4 | -38,2 | -46,6 | -55,0 | -67,6 | -88,5 | -83,0 |
| | 4 | -41,2 | -50,3 | -59,5 | -73,2 | -96,1 | -90,6 |
| phosphonoacetate | 7,4 | -34,8 | -42,7 | -50,7 | -62,5 | -82,3 | 80,7 |
| | 4 | -33,2 | -41,4 | -49,6 | -61,9 | -82,4 | -85,8 |
| phosphonobutyrate | 7,4 | -31,7 | -38,8 | -46,0 | -56,7 | -74,5 | -76,8 |
| | 4 | -24,2 | -30,1 | -36,0 | -44,9 | -59,6 | -74,4 |
| phosphonohexanoate | 7,4 | -29,3 | -35,8 | -42,4 | -52,2 | -68,5 | -73,8 |
| | 4 | -21,7 | -27,1 | -32,4 | -40,3 | -53,6 | -71,4 |
| phosphonooctanoate | 7,4 | -23,2 | -28,2 | -33,3 | -40,8 | -53,3 | -66,2 |

(continued)

| | | average nucleobase use | | | | | |
|---|---|---|---|---|---|---|---|
| | | uniform 2' modified | | | | | 50% mod. |
| | 4 | -15,6 | -19,4 | -23,2 | -28,9 | -38,4 | -63,8 |
| phosphonododecanoate | 7,4 | 0,1 | 0,9 | 1,7 | 2,9 | 4,9 | -37,1 |
| | 4 | 7,7 | 9,7 | 11,7 | 14,8 | 19,9 | -34,7 |
| phosphonocyclohexanoate | 7,4 | -25,6 | -31,2 | -36,8 | -45,3 | -59,3 | -69,2 |
| | 4 | -18,0 | -22,4 | -26,8 | -33,4 | -44,4 | -66,8 |
| phosphono (1-methyl 4 acetyl)cyclohexanoate | 7,4 | -19,8 | -24,0 | -28,2 | -34,5 | -44,9 | -62,0 |
| | 4 | -12,3 | -15,2 | -18,2 | -22,6 | -30,0 | -59,6 |
| phospho-formiate | 7,4 | -33,0 | -40,5 | -47,9 | -59,1 | -77,8 | -78,4 |
| | 4 | -36,1 | -44,4 | -52,6 | -65,0 | -85,6 | -86,1 |
| phosphoacetate | 7,4 | -32,7 | -40,1 | -47,5 | -58,6 | -77,1 | -78,1 |
| | 4 | -36,8 | -45,9 | -54,9 | -68,5 | -91,1 | -90,1 |
| phospho-butyrate | 7,4 | -29,6 | -36,2 | -42,9 | -52,8 | -69,3 | -74,2 |
| | 4 | -22,2 | -27,6 | -33,0 | -41,1 | -54,6 | -71,9 |
| phosho-hexanoate | 7,4 | -25,4 | -30,9 | -36,5 | -44,8 | -58,7 | -68,9 |
| | 4 | -17,8 | -22,1 | -26,5 | -33,0 | -43,8 | -66,5 |
| phospho-octanoic acid | 7,4 | -16,9 | -20,3 | -23,8 | -28,9 | -37,5 | -58,3 |
| | 4 | -9,3 | -11,5 | -13,7 | -17,0 | -22,6 | -55,9 |
| phospho-decanoic acid | 7,4 | -7,9 | -9,0 | -10,1 | -11,8 | -14,6 | -46,7 |
| | 4 | -0,8 | -0,9 | -1,0 | -1,1 | -1,4 | -45,3 |
| phospho-dodecanoic acid | 7,4 | 0,1 | 0,9 | 1,7 | 2,9 | 4,9 | -37,1 |
| | 4 | 7,7 | 9,7 | 11,7 | 14,8 | 19,9 | -34,7 |
| phospho-hexadecanoic acid | 7,4 | 17,1 | 22,2 | 27,2 | 34,8 | 47,5 | -15,8 |
| | 4 | 24,7 | 31,0 | 37,3 | 46,7 | 62.4 | -13,4 |
| Thiophosphonoformiate | 7,4 | -33,4 | -40,5 | -47,7 | -58,4 | -76,3 | -76,9 |
| | 4 | -36,4 | -44,3 | -52,3 | -64,2 | -84,1 | -84,6 |
| Thiophosphonoaceate | 7,4 | -20,0 | -24,2 | -28,4 | -34,8 | -45,3 | -62,2 |
| | 4 | -18,4 | -22,9 | -27,4 | -34,1 | -45,4 | -67,3 |
| Thiophosphonobutyrat | 7,4 | -16,9 | -20,3 | -23,8 | -28,9 | -37,5 | -58,3 |
| | 4 | -9,4 | -11,6 | -13,8 | -17,1 | -22,6 | -55,9 |
| Thiophosphonohexanoate | 7,4 | -14,5 | -17,3 | -20,2 | -24,4 | -31,5 | -55,3 |
| | 4 | -6,9 | -8,5 | -10,2 | -12,6 | -16,6 | -52,9 |
| Thiophosphonocyclohexanoate | 7,4 | -10,8 | -12,7 | -14,6 | -17,5 | -22,3 | -50,7 |
| | 4 | -3,2 | -3,8 | -4,5 | -5,5 | -7,2 | -48,2 |
| Thiophosphono(1 methyl 4 acetyl)cyclohexane | 7,4 | -5,0 | -5,5 | -6,0 | -6,7 | -7,9 | -43,5 |
| | 4 | 2,6 | 3,4 | 4,1 | 5,3 | 7,2 | -41,0 |

The data provided here facilitate the design and optimization of novel oligonucleotides having enhanced cellular penetration properties. The impact of the internucleoside chemistry was analyzed in detail for TEE's of different length and configuration. Statistical oligomers of different length and degree of modification have been analyzed for their pH-dependent logD properties. This data set therefore gives sufficient guidance to apply the thinking of this invention and to construct specific oligomers for practical use.

Analysis for 2' position

TEE's according to (VII) and (VIII) for the enhancement of membrane permeability of an oligonucleotide can also be grafted to the 2' position of the nucleobases and can minimize the overall polarity of the molecule or minimize or revert the increase in polarity that occurs when the oligonucleotide is exposed to low pH. LogD values for a series of related TEE's grafted to 2' position of the nucleoside are reported in the table below. The analysis was done for 20mers of deoxyribonucleotides, wherein the degree of modification was kept at 50%. The total backbone was assumed to be of either phosphodiester type or of PTO type, as indicated in the headings. In detail, the following compounds were analyzed:

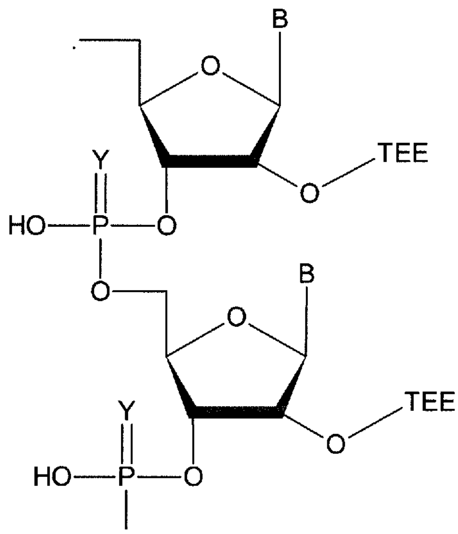

Wherein the formula represents a portion of a longer oligonucleotide and B is any nucleobase selected from adenine, guanine, thymine, cytosine or uracile; Y is O for phoshodiesters or S for phosphorothioates and the TEE is propanoic acid, hexanoic acid, decanoic acid, dodecanoic acid, myristoic acid, palmic acid or 14-oxo-palmic acid, said TEE comprising 3,6,10,12,14,16 or 15 carbon atoms, respectively

| # of C in TEE pH | | phosphodiester | | phosphorothioate | |
|---|---|---|---|---|---|
| | | 4 | 7,4 | 4 | 7,4 |
| | 3 | -91 | -93 | | |
| | 6 | -83 | -85 | -77 | -79 |
| | 10 | -62 | -64 | -56 | -58 |
| | 12 | -51 | -54 | -45 | -47 |
| | 14 | -41 | -43 | -34 | -37 |
| | 16 | -30 | -32 | -24 | -26 |
| *no TEE* | | -91 | *-79,1* | *-83,8* | *-67,3* |

| gain from TEE pH | | phosphodiester | | phosphorothioate | |
|---|---|---|---|---|---|
| | | 4 | 7,4 | 4 | 7,4 |
| | 3 | 0 | -14 | | |
| | 6 | 8 | -6 | 7 | -12 |
| | 10 | 29 | 15 | 28 | 9 |
| | 12 | 40 | 26 | 39 | 20 |
| | 14 | 50 | 36 | 49 | 31 |
| | 16 | 61 | 47 | 60 | 41 |

TEE's comprising six or less carbon atoms do not substantially improve or even reduce the logD of the 20mer

and it becomes apparent from the data that TEE's comprising higher alkyls are needed to substantially reduce the polarity of the oligodeoxyribonucleotide. TEE's in combination with phosphodiester linkages require 10 or more carbon atoms, when used with the more hydrophobic phosphorothioate linkages 8 or more carbon atoms are required to substantially improved the ability of the oligonucleotide to penetrate biological membranes.

The 14-oxo-palmic acid derivative is more polar than its counterpart having a straight alkyl chain free of heteratoms and achieves logD values of about -56 or -50 for the phosphodiester or phosphorothioate backbones, respectively. Use of such derivatives however, might still be advantageous due to the reported structural improvements of substituted 2' ethylenglycolriboses according to Prakash and Bhat (Curr Top Med Chem 2007: 7, 641-649). In more general terms, such structures comprise 12 or more carbon atoms in their TEE and have an ether bond between the second and third terminal carbon atom.

As above, there is structural promiscuity with respect to the configuration of the alkyl chain of the TEE and very similar properties towards a logD can be observed for cycloalkanes, linear or the branched isoalkyl residues. The table below presents more detailed data for the individual configurations of TEE's s grafted onto the 2' position of the nucleoside described above. LogD values have been calculated for pH7.4 and pH4 for abasic nucleoside oligomers comprising 1 to 4 elements. From these initial data, the contribution of a monomer (impact) as well as the virtual starting logD at chain length zero (offset) was calculated. The difference between the impact factors at pH4 and 7.4 reflects the pH sensitive effect of the hydrophilic element of the TEE. Positive values indicate functional TEE's that have the expected hydrophilic-hydrophobic shift mentioned in the description of this invention.

The chemical names and structures have been described above. Further abbraviations in the table are: 2'OMe: 2' O-methyl; 2' MOE: 2'0-methoxyethyl. As above, homogenous modifications with respect to thiolation of the internucleoside linkages were used.

| | | **abasic** | | | | | | |
|---|---|---|---|---|---|---|---|---|
| | | **uniform 2' modified** | | | | | | |
| lenght of oligonucleotide | pH | 1 | 2 | 3 | 4 | impact | offset | (impact) |
| deoxy | 7,4 | -5,6 | -8,1 | -10,6 | -13,2 | -2,5 | -3,0 | 0,0 |
| | 4 | -4.6 | -7,9 | -10,5 | -13,0 | -2,5 | -2,9 | |
| 2' OH | 7,4 | -5,8 | -8,9 | -11,9 | -15,0 | -3,0 | -2,8 | 0,0 |
| | 4 | -5,0 | -8,8 | -11,9 | -14,9 | -3,0 | -2,8 | |
| PTO / DNA | 7,4 | -5,3 | -7,2 | -9,1 | -11,1 | -1,9 | -3,3 | -0,1 |
| | 4 | -3,6 | -6,9 | -9,1 | -11,1 | -2,0 | -3,0 | |
| PTO/RNA | 7,4 | -5,2 | -7,7 | -10,1 | -12,5 | -2,4 | -2,8 | 0,0 |
| | 4 | -4,4 | -7,6 | -10,1 | -12,5 | -2,4 | -2,8 | |
| 2'Ome | 7,4 | -5,3 | -7,7 | -10,1 | -12,5 | -2,4 | -2,9 | 0,0 |
| | 4 | -4,4 | -7,6 | -10,1 | -12,5 | -2,4 | -2,9 | |
| 2' O propyl | 7,4 | -4,5 | -6,2 | -7,8 | -9,5 | -1,6 | -2,9 | 0,0 |
| | 4 | -3,7 | -6,1 | -7,8 | -9,5 | -1,6 | -2,9 | |
| 2'MOE | 7,4 | -6,0 | -9,0 | -12,1 | -15,2 | 3,1 | -2,9 | 0,0 |
| | 4 | -5,1 | -8,9 | -12,1 | -15,2 | 3,1 | -2,9 | |
| 2' o-propanic acid | 7,4 | -7,5 | -11,4 | -15,3 | -19,2 | 3,9 | -3,6 | 1,0 |
| | 4 | -4,8 | -9,1 | -12,3 | -15,2 | -2,9 | -3,5 | |
| 2' O hexanoic acid | 7,4 | -6,7 | -9,9 | -13,1 | -16,2 | 3,2 | -3,6 | 1,0 |
| | 4 | -3,9 | -7,9 | -9,8 | -12,0 | -2,2 | -3,4 | |
| 2' O decanoic acid | 7,4 | -4,6 | -5,6 | -6,7 | -7,7 | -1,0 | -3,6 | 1,0 |
| | 4 | -1,8 | -3,2 | -3,5 | -3,5 | 0,0 | -3,3 | |
| 2' O dodecanoic acid | 7,4 | -3,5 | -3,5 | -3,5 | -3,5 | 0,0 | -3,6 | 1,0 |
| | 4 | -0,7 | -1,0 | -0,3 | 0,8 | 1,0 | -3,3 | |
| 2' 0 myristoic acid | 7,4 | -2,5 | -1,4 | -0,3 | 0,8 | 1,1 | -3,6 | 1,0 |
| | 4 | 0,3 | 1,1 | 2,9 | 5,0 | 2,1 | -3,3 | |
| 2'O palmic acid | 7,4 | -1,4 | 0,7 | 2,9 | 5,1 | 2,2 | -3,6 | 1,0 |

(continued)

| | | abasic | | | | | | |
|---|---|---|---|---|---|---|---|---|
| | | uniform 2' modified | | | | | | |
| lenght of oligonucleotide | pH | 1 | 2 | 3 | 4 | impact | offset | (impact) |
| | 4 | 1,4 | 3,2 | 6,1 | 9,3 | 3,2 | -3,3 | |
| 2' O-(14 oxo)-hexadecanoic acid | 7,4<br>4 | -3,4<br>-0,6 | -3,2<br>-0,7 | -3,0<br>0,3 | -2,8<br>1,5 | 0,2<br>1,2 | -3,6<br>-3,3 | 1,0 |
| | | -6,9<br>-4,2 | -10,2<br>-7,9 | -13,5<br>-10,5 | -16,8<br>-12,8 | -3,3<br>-2,3 | -3,6<br>-3,5 | 1,0 |
| 2' O hexanoic acid, PTO | 7,4<br>4 | -6,1<br>-3,3 | -9,0<br>-6,2 | -11,2<br>-8,0 | -13,8<br>-9,6 | -2,6<br>-1,6 | -3,6<br>-3,3 | 1,0 |
| 2' O decanoic acid, PTO | 7,4<br>4 | -4,0<br>-1,2 | -4,4<br>-2,0 | -4,9<br>-1,6 | -5,3<br>-1,1 | -0,4<br>0,6 | -3,6<br>3,4 | 1,0 |
| 2'O dodecanoic acid, PTO | 7,4<br>4 | -2,9<br>-0,1 | -2,3<br>0,2 | -1,7<br>1,6 | -1,0<br>3,2 | 0,6<br>1,6 | -3,6<br>-3,3 | 1,0 |
| 2' O myristic acid, PTO | 7,4<br>4 | -1,9<br>0,9 | -0,2<br>2,3 | 1,5<br>4,7 | 3,2<br>7,4 | 1,7<br>2,7 | -3,6<br>-3,3 | 1,0 |
| 2' O palmic acid, PTO | 7,4<br>4 | -0,8<br>2,0 | 1,9<br>4,4 | 4,7<br>7,9 | 7,5<br>11,7 | 2,8<br>3,8 | -3,6<br>-3,4 | 1,0 |
| 2' O-(14 oxo)-hexadecanoic acid | 7,4<br>4 | -2,8<br>0,0 | -2,0<br>0,5 | -1,2<br>2,1 | -0,4<br>3,9 | 0,8<br>1,8 | -3,6<br>-3,3 | 1,0 |

Starting from this set of data, higher oligomers were analyzed that also incorporated average nucleobases. For the latter, the logD coefficients reported above (-1.3 at pH7.4 and -1.7 at pH4) were used. The table below reports data for 8mers to 20mers that comprise homogenously 2' TEE modified nucleosides and for 20mers with about 50% degree of modification. The specific appearance of the modified nucleobases in the sequence of nucleotides is of no substantial relevance to the logD values reported here, thus the modified nucleosides might be dispersed statistically throughout the oligomer, they might concentrate on one end, or gapmers with modified flanks and unmodified central regions may be formed.

| | | average nucleobase use | | | | | |
|---|---|---|---|---|---|---|---|
| | | uniform 2' modified | | | | | 50% mod. |
| lenght of oligonucleotide | pH | 8 | 10 | 12 | 15 | 20 | 20 |
| deoxy | 7,4<br>4 | -33,5<br>-36,4 | -41,1<br>-44,8 | -48,7<br>-53,2 | -60,2<br>-65,8 | -79,2<br>-86,8 | -79,2<br>-86,8 |
| 2'OH | 7,4<br>4 | -37,3<br>-40,3 | -45,9<br>-49,7 | -54,5<br>-59,1 | -67,4<br>-73,2 | -89,0<br>-96,7 | -84,2<br>-91,8 |
| PTO/DNA | 7,4<br>4 | -28,9<br>-32,4 | -35,3<br>-39,8 | -41,7<br>-47,1 | -51,3<br>-58,1 | -67,3<br>-76,5 | -73,1<br>-81,6 |
| PTO/RNA | 7,4<br>4 | -32,5<br>-35,5 | -39,9<br>-43,7 | -47,3<br>-51,9 | -58,4<br>-64,2 | -77,0<br>-84,7 | -78,2<br>-85,8 |
| 2'Ome | 7,4<br>4 | -32,3<br>-35,4 | -39,7<br>-43,5 | -47,1<br>-51,7 | -58,1<br>-63,9 | -76,5<br>-84,2 | -77,9<br>-85,5 |
| 2' O propyl | 7,4<br>4 | -26,2<br>-29,3 | -32,0<br>-35,9 | -37,8<br>-42,4 | -46,6<br>-52,3 | -61,1<br>-68,8 | -70,2<br>-77,8 |
| 2'MOE | 7,4 | -37,6 | -46,3 | -55,0 | -68,0 | -89,7 | -84,5 |

(continued)

| | | average nucleobase use | | | | | |
|---|---|---|---|---|---|---|---|
| | | uniform 2' modified | | | | | 50% mod. |
| lenght of oligonucleotide | pH | 8 | 10 | 12 | 15 | 20 | 20 |
| | 4 | -40,7 | -50,1 | -59,6 | -73,7 | -97,4 | -92,1 |
| 2' o-propanic acid | 7,4<br>4 | -45,0<br>-40,2 | -55,4<br>-49,3 | -65,7<br>-58,5 | -81,3<br>-72,3 | -107,2<br>-95,2 | -92,9<br>-90,7 |
| 2' O hexanoic acid | 7,4<br>4 | -39,0<br>-33,9 | -47,8<br>-41,5 | -56,6<br>-49,1 | -69,9<br>-60,6 | -92,0<br>-79,7 | -85,3<br>-83,0 |
| 2' O decanoic acid | 7,4<br>4 | -22,0<br>-16,9 | -26,6<br>-20,3 | -31,2<br>-23,7 | -38,1<br>-28,8 | -49,6<br>-37,2 | -64,1<br>-61,8 |
| 2' O dodecanoic acid | 7,4<br>4 | -13,5<br>-8,4 | -16,0<br>-9,7 | -18,5<br>-10,9 | -22,2<br>-12,8 | -28,4<br>-16,0 | -53,5<br>-51,2 |
| 2' O myristoic acid | 7,4<br>4 | -5,0<br>0.1 | -5,3<br>0,9 | -5,6<br>1,8 | -6,2<br>3,1 | -7,0<br>5,2 | -42,8<br>-40,6 |
| 2'O palmic acid | 7,4<br>4 | 3,5<br>8,6 | 5,3<br>11,6 | 7,1<br>14,6 | 9,8<br>19,1 | 14,2<br>26,6 | -32,2<br>-29,9 |
| 2' O-(14 oxo)-hexadecanoic acid | 7,4<br>4 | -12,1<br>-7,0 | -14,2<br>-7,9 | -16,3<br>-8,8 | -19,5<br>-10,2 | -24,8<br>-12,4 | -51,7<br>-49,4 |
| | | -40,1<br>-35,3 | -49,2<br>-43,3 | -58,3<br>-51,2 | -72,0<br>-63,1 | -94,8<br>-83,0 | -86,7<br>-84,6 |
| 2' O hexanoic acid, PTO | 7,4<br>4 | -34,2<br>-29,1 | -41,8<br>-35,5 | -49,4<br>-41,9 | -60,9<br>-51,6 | -80,0<br>-67,6 | -79,3<br>-77,0 |
| 2' O decanoic acid, PTO | 7,4<br>4 | -17,2<br>-12,0 | -20,6<br>-14,2 | -24,0<br>-16,4 | -29,1<br>-19,6 | -37,6<br>-25,1 | -58,1<br>-55,7 |
| 2'0 dodecanoic acid, PTO | 7,4<br>4 | -8,6<br>-3,5 | -9,9<br>-3,6 | -11,2<br>-3,6 | -13,1<br>-3,7 | -16,2<br>-3,8 | -47,4<br>-45,1 |
| 2' O myristic acid, PTO | 7,4<br>4 | -0,1<br>4,9 | 0,7<br>7,0 | 1,6<br>9,1 | 2,9<br>12,2 | 5,0<br>17,4 | -36,8<br>-34,5 |
| 2' O palmic acid, PTO | 7,4<br>4 | 8,3<br>13,5 | 11,3<br>17,7 | 14,3<br>21,9 | 18,8<br>28,2 | 26,2<br>38,7 | -26,2<br>-23,8 |
| 2' O-(14 oxo)-hexadecanoic acid | 7,4<br>4 | -7,3<br>-2,2 | -8,2<br>-1,9 | -9,1<br>-1,6 | -10,5<br>-1,2 | -12,8<br>-0,4 | -45,7<br>-43,4 |

The data provided here facilitate the design and optimization of novel, 2' modified oligonucleotides having enhanced cellular penetration properties. The impact of the internucleoside chemistry was analyzed in detail for TEE's of different length and configuration. Statistical oligomers of different length and degree of modification have been analyzed for their pH-dependent logD properties. This data set therefore gives sufficient guidance to modify the thinking of this invention and to construct specific oligomers for practical use.

As a further illustration, the analysis of oligonucleotides was extended to non-phosphoribose compounds to which TEE's as specified in (VII) or (VIII) can be attached. LogD values for a series of related TEE's grafted to the secondary amine or TEE's s grafted onto the methylene bridge in a peptide nucleic acid (PNA) backbone are reported in the table below. In detail, the following compounds were analyzed:

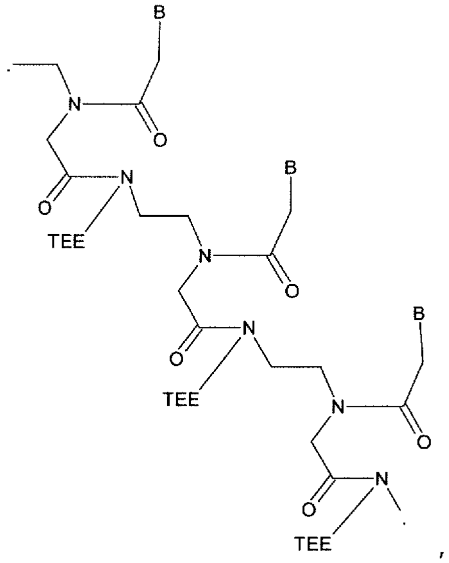

wherein the formula shows a fragment of a longer oligonucleotide, B represents any of the nucleobases adenine, guanine, cytosine, thymine or uracile and hexanoic acid, octanoic acid, decanoic acid, dodecanoic acid, 4-methyl-cyclohexyl-1-carboxylic acid or 8-oxo-nonaoic acid were used as specific representations of TEE's.

In addition, the following structures have been analyzed:

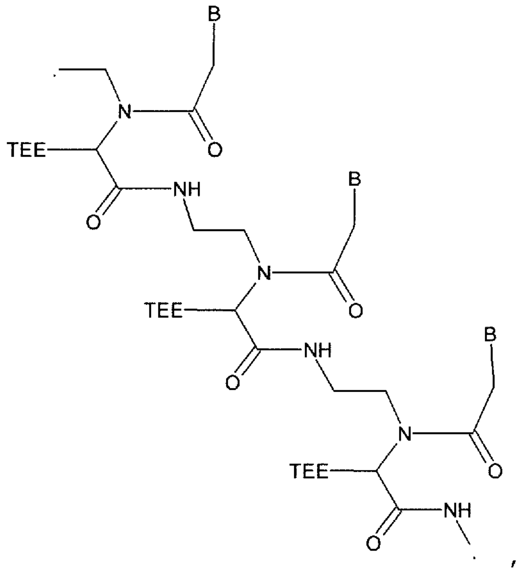

wherein the formula shows a fragment of a longer oligonucleotide, B represents any of the nucleobases adenine, guanine, cytosine, thymine or uracile and octanoic acid is a specific representation of a TEE's.

The table below presents more detailed data for the individual configurations of TEE's grafted onto both PNA structures. LogD values have been calculated for pH7.4 and pH4 for abasic oligomers. From these initial data, the contribution of a monomer (impact) as well as the virtual starting logD at chain length zero (offset) was calculated. The difference between the impact factors at pH4 and 7.4 reflects the pH sensitive effect of the hydrophilic element of the TEE. Positive values indicate functional TEE's that have the expected hydrophilic-hydrophobic shift mentioned in the description of this invention.

| | | abasic | | | | | | |
|---|---|---|---|---|---|---|---|---|
| | | uniform 2' modified | | | | | | |
| lenght of oligonucleotide | pH | 1 | 2 | 3 | 4 | impact | offset | (impact) |
| doexy | 7,4<br>4 | -5,6<br>-4,6 | -8,1<br>-7,9 | -10,6<br>-10,5 | -13,2<br>-13,0 | -2,5<br>-2,5 | -3,0<br>-2,9 | 0,0 |
| 2' OH, | 7,4<br>4 | -5,8<br>-5,0 | -8,9<br>-8,8 | -11,9<br>-11,9 | -15,0<br>-14,9 | -3,0<br>-3,0 | -2,8<br>-2,8 | 0,0 |
| PTO/ DNA | 7,4<br>4 | -5,3<br>-3,6 | -7,2<br>-6,9 | -9,1<br>-9,1 | -11,1<br>-11,1 | -1,9<br>-2,0 | -3,3<br>-3,0 | -0,1 |
| PTO/RNA | 7,4<br>4 | -5,2<br>-4,4 | -7,7<br>-7,6 | -10,1<br>-10,1 | -12,5<br>-12,5 | -2,4<br>-2,4 | -2,8<br>-2,8 | 0,0 |
| 2'Ome | 7,4<br>4 | -5,3<br>-4,4 | -7,7<br>-7,6 | -10,1<br>-10,1 | -12,5<br>-12,5 | -2,4<br>-2,4 | -2,9<br>-2,9 | 0,0 |
| 2' O propyl | 7,4<br>4 | -4,5<br>-3,7 | -6,2<br>-6,1 | -7,8<br>-7,8 | -9,5<br>-9,5 | -1,6<br>-1,6 | -2,9<br>-2,9 | 0,0 |
| 2'MOE | 7,4<br>4 | -6,0<br>-5,1 | -9,0<br>-8,9 | -12,1<br>-12,1 | -15,2<br>-15,2 | 3,1<br>3,1 | -2,9<br>-2,9 | 0,0 |
| | | | | | | | | |
| PNA unmodified | 7,4<br>4 | -1,3<br>-1,3 | -3,3<br>-3,3 | -5,2<br>-5,2 | | -1,9<br>-1,9 | 0,6<br>0,6 | 0,0 |
| PNA, N-hexanoic acid | 7,4<br>4 | -3,1<br>-0,6 | -5,7<br>-1,3 | -7,5<br>-1,9 | | -1,7<br>-0,7 | -2,3<br>0,1 | 1,0 |
| PNA, N-octanoic acid | 7,4<br>4 | -2,0<br>0,5 | -3,6<br>0,9 | -4,3<br>1,3 | | -0,7<br>0,4 | -2,3<br>0,1 | 1,0 |
| PNA,N-decanoicacid | 7,4<br>4 | -1,0<br>1,6 | -1,5<br>3,0 | -1,1<br>4,5 | | 0,4<br>1,4 | -2,3<br>0,1 | 1,0 |
| PNA, N-dodecanoic acid | 7,4<br>4 | 0,1<br>2,6 | 0,7<br>5,1 | 2,1<br>7,6 | | 1,5<br>2,5 | -2,3<br>0,1 | 1,0 |
| PNA, N-(4methyl)cydohexan-1-oic acid | 7,4<br>4 | -2,5<br>-0,1 | -4,8<br>-0,4 | -6,2<br>-0,6 | | -1,4<br>-0,2 | -2,0<br>0,1 | 1,2 |
| PNA, N-(8-oxo) nonaoic acid | 7,4<br>4 | -2,4<br>0,1 | -4,1<br>0,4 | -4,8<br>0,7 | | -0,7<br>0,3 | -2,6<br>-0,2 | 1,0 |
| PNA, C-octanoic acid | 7,4<br>4 | -2,0<br>0,6 | -3,9<br>0,6 | | | -2,0<br>0,0 | 0,0<br>0,6 | 2,0 |

Starting from this set of data, higher oligomers were analyzed that also incorporated average nucleobases. For the latter, the logD coefficients reported above (-1.3 at pH7.4 and -1.7 at pH4) were used. The table below reports data for 8mers to 20mers that comprise homogenously modified internucleoside linkages and for 20mers with about 50% degree of modification. The specific appearance in the sequence of internucleoside linkages is of no substantial relevance to the logD values reported here, the modified linkages might be dispersed statistically throughout the oligomer, they might concentrate on one end, or gapmers with modified flanks and unmodified central regions may be formed.

| | | **average nucleobase use** | | | | | |
|---|---|---|---|---|---|---|---|
| | | **uniform 2' modified** | | | | | **50% mod.** |
| **lenght of oligonucleotide** | pH | 8 | 10 | 12 | 15 | 20 | 20 |
| **deoxy** | 7,4 | -33,5 | -41,1 | -48,7 | -60,2 | -79,2 | -79,2 |

(continued)

| | | average nucleobase use | | | | | |
|---|---|---|---|---|---|---|---|
| | | uniform 2' modified | | | | | 50% mod. |
| lenght of oligonucleotide | pH | 8 | 10 | 12 | 15 | 20 | 20 |
| | 4 | -36,4 | -44,8 | -53,2 | -65,8 | -86,8 | -86,8 |
| 2'OH | 7,4 | -37,3 | -45,9 | -54,5 | -67,4 | -89,0 | -84,2 |
| | 4 | -40,3 | -49,7 | -59,1 | -73,2 | -96,7 | -91,8 |
| PTO / DNA | 7,4 | -28,9 | -35,3 | -41,7 | -51,3 | -67,3 | -73,1 |
| | 4 | -32,4 | -39,8 | -47,1 | -58,1 | -76,5 | -81,6 |
| PTO/RNA | 7,4 | -32,5 | -39,9 | -47,3 | -58,4 | -77,0 | -78,2 |
| | 4 | -35,5 | -43,7 | -51,9 | -64,2 | -84,7 | -85,8 |
| 2'Ome | 7,4 | -32,3 | -39,7 | -47,1 | -58,1 | -76,5 | -77,9 |
| | 4 | -35,4 | -43,5 | -51,7 | -63,9 | -84,2 | -85,5 |
| 2'O propyl | 7,4 | -26,2 | -32,0 | -37,8 | -46,6 | -61,1 | -70,2 |
| | 4 | -29,3 | -35,9 | -42,4 | -52,3 | -68,8 | -77,8 |
| 2'MOE | 7,4 | -37,6 | -46,3 | -55,0 | -68,0 | -89,7 | -84,5 |
| | 4 | -40,7 | -50,1 | -59,6 | -73,7 | -97,4 | -92,1 |
| | | | | | | | |
| PNA unmodified | 7,4 | -24,8 | -31,2 | -37,5 | -47,0 | -62,9 | -72,8 |
| | 4 | -27,9 | -35,0 | -42,1 | -52,8 | -70,6 | -80,4 |
| PNA, N-hexanoic acid | 7,4 | -26,2 | -32,1 | -38,1 | -47,0 | -61,9 | -70,9 |
| | 4 | -18,6 | -23,3 | -28,0 | -35,0 | -46,8 | -68,3 |
| PNA, N-octanoic acid | 7,4 | -17,7 | -21,6 | -25,4 | -31,2 | -40,9 | -60,4 |
| | 4 | -10,1 | -12,6 | -15,2 | -19,0 | -25,4 | -57,6 |
| PNA, N-decanoic acid | 7,4 | -9,2 | -11,0 | -12,7 | -15,3 | -19,7 | -49,8 |
| | 4 | -1,6 | -2,0 | -2,5 | -3,1 | -4,2 | -47,0 |
| PNA, N-dodecanoic acid | 7,4 | -0,7 | -0,3 | 0,1 | 0,7 | 1,7 | -39,1 |
| | 4 | 6,9 | 8,6 | 10,3 | 12,8 | 17,0 | -36,4 |
| PNA, N-(4methyl) cyclohexan-1-oic acid | 7,4 | -23,3 | -28,6 | -33,9 | -41,9 | -55,2 | -67,7 |
| | 4 | -15,0 | -18,8 | -22,6 | -28,3 | -37,8 | -63,8 |
| PNA, N-(8-oxo) nonaoic acid | 7,4 | -18,7 | -22,7 | -26,7 | -32,8 | -42,8 | -61,2 |
| | 4 | -11,2 | -13,9 | -16,7 | -20,8 | -27,7 | -58,6 |
| PNA, C-octanoic acid | 7,4 | -25,9 | -32,4 | -38,9 | -48,6 | -64,8 | -73,5 |
| | 4 | -12,7 | -16,0 | -19,3 | -24,3 | -32,6 | -61,4 |

The charge-neutral backbone of the PNA, although being responsible for a higher binding affinity of the oligonucleotide and better specificity compared to internucleoside linkages based on phosphorus, has no substantial difference in their logD to its natural counterpart. This is in line with the observation that PNA oligomers cannot easily penetrate cells and need transfecting agents as any other oligonucleotide. Modification with TEE's is changing the picture and oligonucleotides comprising TEE's with 8 or more carbon atoms have enhanced cell permeability.

The use of TEE's in combination with oligonucleotides is not limited to covalent attachments within the structure of the oligonucleotide and the inventive concept also applies to physically attached moieties and to conjugates of oligonucleotides with multivalent TEE's. The abovementioned polycations offer ample opportunity for derivatization and formation of multivalent TEE's. Alkylation of a polycations' nitrogen group keeps its charged state, thus enabling electrostatic binding between the TEE carrying polycation and the oligonucleotide. In addition, charge neutralization between the polycation and the internucleoside linkages further reduces the logD. In a specific example a dispermin was coupled from spermin monomers using a butylene linker yielding a linear octa-amine with alternating C4 and C3 spacers. TEE's s can be added to that skeleton using w-halogen-a-carboxylic acids leading to the molecular structure below

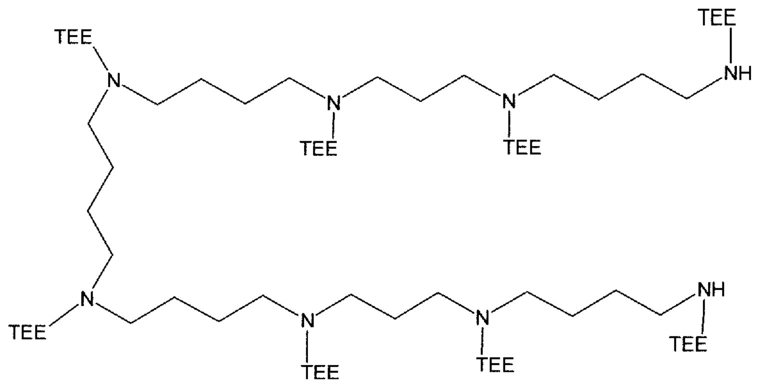

wherein TEE is hydrogen or a TEE as defined above in (VII) or (VIII) and one or more TEE's are present on the polycation.

Higher polycations can be used as well and it is possible to apply the teachings of this invention towards TEE-substituted trispermines tetraspermines and oligospermines. The spacing between the amino groups in the polycation can be varied as well and the individual nitrogens may be 3 to 8 bonds apart from each other. In preferred configurations, this distance is between 3 and 6 bonds on average. It is known to the skilled artisan that the average spacing in spermin of 3.5 C-C units equals the distance between the charged groups on a oligonucleotide backbone, thus providing a good fit for the electrostatic interactions between this polycationic structure and the internucleoside linkages. The polycations may comprise more than 4 and less than 1000 charged elements. In some preferred aspects, the polycations are linear polyamines with more than 4 and less than 20 charged groups. In more preferred aspects, such linear polyamines have between 6 and 12 charged groups and the abovementioned conditions for the spacing of these groups apply, that is, these preferred polyamines have charged groups being separated by 3 to 6 bonds.

Also, other polycations such as linear or branched polyethylenimine can be derivatized with the TEE's s disclosed above and can be used as carrier systems for oligonucleotides.

It is of course possible to design mixed forms of oligonucleotide-polycation complexes, wherein the polycation is covalently bound to the oligonucleotide via linking groups. The structure below presents an example of such comjugates that should illustrate this aspect of the invention:

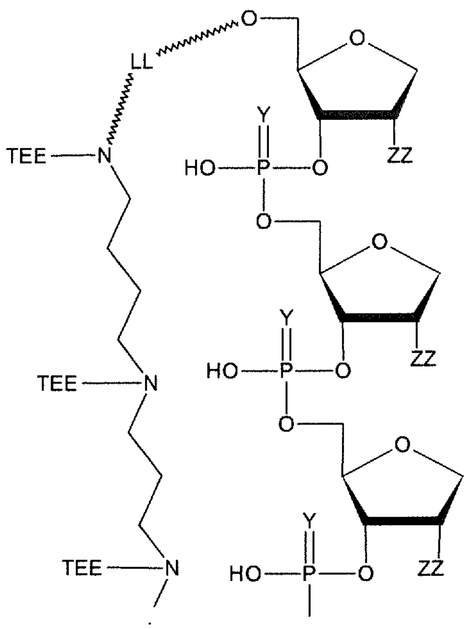
,

wherein the formula depicts a fragment of an oligonucleotide further comprising a polycation/TEE extension and TEE is independently absent or any of the structures defined in (VII) or (VIII); B is any nucleobase selected from adenine, guanine, thymine, cytosine or uracile; Y is O for phoshodiesters or S for phosphorothioates; ZZ can be H, OH, O-methyl, O-methoxyethyl, F, amino, thio and LL is absent or a covalent linker.

LogD values for a series of related TEE's grafted onto polycations and further conjugated to a phosphoribose

backbone are reported in the table below. The analysis was started for abasic oligomers of phosphoribose conjugated to polycations having the identical number of charged groups. The backbone was assumed to be of either phosphodiester type or of PTO type, as indicated in the headings and the TEE was absent, hexanoic acid or dodecanoic acid. In detail, the following compounds were analyzed:

| | | abasic | | | | | | |
|---|---|---|---|---|---|---|---|---|
| | | uniform 2' modified | | | | | | |
| **lenght of oligonucleotide** | pH | 1 | 2 | 3 | 4 | impact | offset | η (impact) |
| **deoxy** | 7,4 | -5,6 | -8,1 | -10,6 | -13,2 | -2,5 | -3,0 | 0,0 |
| | 4 | -4,6 | -7,9 | -10,5 | -13,0 | -2,5 | -2,9 | |
| 2'OH | 7,4 | -5,8 | -8,9 | -11,9 | -15,0 | 3,0 | -2,8 | 0,0 |
| | 4 | -5,0 | -8,8 | -11,9 | -14,9 | -3,0 | -2,8 | |
| PTO / DNA | 7,4 | -5,3 | -7,2 | -9,1 | -11,1 | -1,9 | -3,3 | -0,1 |
| | 4 | -3,6 | -6,9 | -9,1 | -11,1 | -2,0 | -3,0 | |
| PTO/RNA | 7,4 | -5,2 | -7,7 | -10,1 | -12,5 | -2,4 | -2,8 | 0,0 |
| | 4 | -4,4 | -7,6 | -10,1 | -12,5 | -2,4 | -2,8 | |
| 2'Ome | 7,4 | -5,3 | -7,7 | -10,1 | -12,5 | -2,4 | -2,9 | 0,0 |
| | 4 | -4,4 | -7,6 | -10,1 | -12,5 | -2,4 | -2,9 | |
| 2' O propyl | 7,4 | -4,5 | -6,2 | -7,8 | -9,5 | -1,6 | -2,9 | 0,0 |
| | 4 | -3,7 | -6,1 | -7,8 | -9,5 | -1,6 | -2,9 | |
| 2'MOE | 7,4 | -6,0 | -9,0 | -12,1 | -15,2 | 3,1 | -2,9 | 0,0 |
| | 4 | -5,1 | -8,9 | -12,1 | -15,2 | 3,1 | -2,9 | |
| | | | | | | | | |
| phosphodeoxyribose, PDE 1:1 aminoconjugate | 7,4 | -5,1 | -6,5 | -8,1 | -9,5 | -1,4 | -4,1 | 0,0 |
| | 4 | -5,1 | -6,5 | -8,1 | -9.5 | -1,4 | -4,1 | |
| phosphodeoxyribose, PTO 1:1 aminoconjugate | 7,4 | -4,5 | -5,3 | -6,3 | -7,1 | -0,8 | -4,1 | 0,0 |
| | 4 | -4,5 | -5,3 | -6,3 | -7,1 | -0,8 | -4,1 | |
| phosphoribose, PTO 1:1 amino+1 TEE C6 | 7,4 | -4,0 | -5,3 | -6,8 | -8,3 | -1,6 | -2,1 | 0,4 |
| | 4 | 3,1 | -3,9 | -4,9 | -6,1 | -1,2 | -1,4 | |
| phosphoribose, PTO 1:1 amino+full TEE C6 | 7,4 | -4,0 | -5,2 | -7,0 | | -1,7 | -1,8 | 1,7 |
| | 4 | -3.1 | -2,9 | -3,0 | | -0.1 | -2,7 | |
| phosphoribose, PTO 1:1 amino+1 TEE C12 | 7,4 | -0,9 | -2,1 | -4,1 | -5,1 | -1,0 | -1,1 | 0,3 |
| | 4 | 0,1 | -0,7 | -2,2 | -2,9 | -0,8 | 0,1 | |
| phosphoribose, PTO 1:1 amino+2 TEE C12 | 7,4 | | 1,1 | -0,4 | -1,7 | -1,2 | 3,3 | 0,5 |
| | 4 | | 3,5 | 2,4 | 1,7 | -0,8 | 4,7 | |
| phosphoribose, PTO 1:1 amino, full TEE C12 | 7,4 | -0,9 | 1,1 | 2,6 | | 1,5 | -1,8 | 1,6 |
| | 4 | 0,1 | 3,5 | 6,6 | | 3,1 | -2,7 | |

It becomes apparent from the data that the salt formation between the internucleoside phosphate and the amino groups of the polycation reduces the polarity of the complex to some extent. However, this effect is independent from the pH and the impact factors for both pH values are identical. This situation changes upon introduction of the first TEE which renders the compounds less hydrophilic at low pH. A particular strong effect is observed upon full complexation of all the phosphate or phosphothioate groups with a TEE modified amino group, the impact factors at pH7.4 and pH 4 are then different by about 1.7. Extension of such observations towards the design of larger oligomers is given below:

| | | average nucleobase use | | | | | |
|---|---|---|---|---|---|---|---|
| | | uniform 2' modified | | | | | 50% mod. |
| lenght of oligonucleotide | pH | 8 | 10 | 12 | 15 | 20 | 20 |
| deoxy | 7,4 | -33,4 | -41,1 | -48,7 | -60,1 | -79,1 | -82,1 |
| | 4 | -37,6 | -46,5 | -55,4 | -68,7 | -91,0 | -93,0 |
| 2'OH | 7,4 | -37,3 | -45,9 | -54,5 | -67,5 | -89,1 | -87,0 |
| | 4 | -41,6 | -51,5 | -61,4 | -76,3 | -101,1 | -98,0 |
| PTO / DNA | 7,4 | -28,9 | -35,3 | -41,7 | -51,3 | -67,3 | -76,4 |
| | 4 | -34,4 | -42,7 | -50,9 | -63,2 | -83,8 | -89,2 |
| PTO/RNA | 7,4 | -32,5 | -39,9 | -47,3 | -58,4 | -77,0 | -80,9 |
| | 4 | -36,7 | -45,5 | -54,2 | -67,2 | -89,0 | -91,9 |
| 2' Ome | 7,4 | -32,3 | -39,7 | -47,1 | -58,1 | -76,5 | -80,7 |
| | 4 | -36,6 | -45,3 | -53,9 | -66,9 | -88,5 | -91,8 |
| 2'O propyl | 7,4 | -26,2 | -32,0 | -37,9 | -46,6 | -61,2 | -73,1 |
| | 4 | -30,5 | -37,6 | -44,7 | -55,4 | -73,2 | -84,1 |
| 2'MOE | 7,4 | -37,6 | -46,3 | -54,9 | -67,9 | -89,6 | -87,3 |
| | 4 | -41,9 | -51,9 | -61,9 | -76,8 | -101,8 | -98,4 |
| phosphodeoxyribose, PDE | 7,4 | -25,7 | -31,3 | -36,8 | -45,1 | -59,0 | -66,6 |
| 1:1 aminoconjugate | 4 | -28,8 | -35,1 | -41,4 | -50,9 | -66,7 | -77,8 |
| phosphodeoxyribose, PTO | 7,4 | -20,9 | -25,3 | -29,6 | -36,1 | -47,0 | -60,6 |
| 1:1 aminoconjugate | 4 | -24,0 | -29,1 | -34,2 | -41,9 | -54,7 | -71,8 |
| phosphoribose, PTO | 7,4 | -24,6 | -30,2 | -35,8 | -44,1 | -58,1 | -65,5 |
| 1:1 amino+1 TEE C6 | 4 | -23,8 | -29,4 | -34,9 | -43,2 | -57,1 | -72,0 |
| phosphoribose, PTO | 7,4 | -24,4 | -29,8 | -35,3 | -43,5 | -57,2 | -65,2 |
| 1:1 amino+full TEE C6 | 4 | -15,8 | -19,0 | -22,2 | -27,0 | -34,9 | -61,7 |
| phosphoribose, PTO | 7,4 | -21,3 | -26,8 | -32,3 | -40,5 | -54,3 | -62,2 |
| 1:1 amino+1 TEE C12 | 4 | -20,4 | -25,8 | -31,2 | -39,4 | -52,9 | -68,5 |
| phosphoribose, PTO | 7,4 | -17,5 | -22,8 | -28,2 | -36,2 | -49,5 | -63,9 |
| 1:1 amino+2 TEE C12 | 4 | -15,2 | -20,3 | -25,5 | -33,1 | -45,9 | -66,8 |
| phosphoribose, PTO | 7,4 | 1,2 | 2,1 | 3,0 | 4,4 | 6,7 | -33,2 |
| 1:1 amino, full TEE C12 | 4 | 9,7 | 12,9 | 16,1 | 21,0 | 29,0 | -29,7 |

All TEE modified conjugates minimize the pH induced decrease of logD that is observed for the parent compounds like DNA or RNA. For these, the logD is about 10 units lower at pH 4 than at pH7.4 for a 20mer oligonucleotide. Presence of TEE's and increase of logD at pH4 are positively correlated and with sufficiently high numbers of TEE's being present, the logD at pH4 may even be less negative than the logD at pH7.4. Partial coverage of about 50% of the oligonucleotide with the polycation-TEE conjugate may also be sufficient to render the oligonucleotide more membrane permeable, as observed for conjugates with dodecanoic acid.

Higher polycations can be used as well and it is possible to extend the teachings of this invention towards TEE-substituted trispermins, tetraspermins, and oligospermins. The spacing between the amino groups in the polycation can be varied as well and the individual nitrogens may be 3 to 8 bonds apart from each other. In preferred configurations, this distance is between 3 and 6 bonds on average. It is known to the skilled artisan that the average spacing in spermin of 3.5 C-C units equals the distance between the charged groups on a oligonucleotide backbone, thus providing a good fit for the electrostatic interactions between this polycationic structure and the internucleoside linkages. The polycations may comprise more than 4 and less than 20 charged groups. In preferred aspects, such linear polyamines have between 6 and 12 charged groups and the abovementioned conditions for the spacing of these groups apply, that is, these preferred polyamines have charged groups being separated by 3 to 6 bonds.

Also, other polycations such as linear or branched polyethylenimine can be derivatized with the TEE's s disclosed above and can be used as carrier systems for oligonucleotides

Use of TEE's for the improvement of transfection is conceptually different from a mere hydrophobic modification of the nucleic acids. Hydrophobic modification has been disclosed e.g. by Letsinger et al. in US 4958013 or Proc. Natl. Acad. Sci., 86, 6553-6556, 1989 or by Manoharan et al. in US 6,153,737 and US 6,753,423 in combination with single stranded oligonucleotides and was also used to deliver siRNA in vivo (Soutschek et al., Nature, 432(7014), 173-178, 2004). A mere hydrophobic modification does not respond to changes in pH of the environment and is therefore different from the TEE's of the invention.

Practicing the invention and use of TEE's is not limited to otherwise unmodified oligonucleotides and TEE's can be grafted on many different nucleotides, oligonucleotides or nucleic acids or polynucleic acids.

The TEE's s described in the present invention are well suited for modify nucleic acid-based drugs such for example as oligonucleotides, polynucleotides or DNA plasmids. These drugs may be classified into nucleic acids that encode one or more specific sequences for proteins, polypeptides or RNAs and into oligonucleotides that can specifically regulate protein expression levels or affect the protein structure through interference with splicing, artificial truncation and others.

In some embodiments of the present invention, therefore, the nucleic acid-based therapeutic may comprise a nucleic acid that is capable of being transcribed in a vertebrate cell into one or more RNAs, which RNAs may be mRNAs, shRNAs, miRNAs or ribozymes, wherein such mRNAs code for one or more proteins or polypeptides. Such nucleic acid therapeutics may be circular DNA plasmids, linear DNA constructs, like MIDGE vectors (Minimalistic Immunogenically Defined Gene Expression) as disclosed in WO 98/21322 or DE 19753182, or mRNAs ready for translation (e.g., EP 1392341).

In another embodiment of the invention, oligonucleotides may be used that can target existing intracellular nucleic acids or proteins. Said nucleic acids may code for a specific gene, such that said oligonucleotide is adapted to attenuate or modulate transcription, modify the processing of the transcript or otherwise interfere with the expression of the protein. The term "target nucleic acid" encompasses DNA encoding a specific gene, as well as all RNAs derived from such DNA, being pre-mRNA or mRNA. A specific hybridisation between the target nucleic acid and one or more oligonucleotides directed against such sequences may result in an inhibition or modulation of protein expression. To achieve such specific targeting, the oligonucleotide should suitably comprise a continuous stretch of nucleotides that is substantially complementary to the sequence of the target nucleic acid.

Oligonucleotides fulfilling the abovementioned criteria may be built with a number of different chemistries and topologies. The oligonucleotides may comprise naturally occurring or modified nucleosides comprising but not limited to DNA, RNA, locked nucleic acids (LNA's), 2' O-methyl RNA (2' Ome), 2' O-methoxyethyl RNA (2'MOE) in their phosphate or phosphothioate forms or Morpholinos or peptide nucleic acids (PNA's).
Oligonucleotides may be single stranded or double stranded.

The mechanisms of action of oligonucleotides may vary and might comprise effects on splicing, transcription, nuclear-cytoplasmic transport and translation, amongst others.

In a preferred embodiment of the invention single stranded oligonucleotides may be used including but are not limited to DNA-based oligonucleotides, locked nucleic acids, 2' -modified oligonucleotides and others, commonly known as antisense oligonucleotides. Backbone or base or sugar modifications may include but are not limited to Phosphothioate DNA (PTO), 2'0-methyl RNA (2' Ome), 2' fluororibose or 2' fluoroarabinose, 2' O-methoxyethyl-RNA (2'MOE), peptide nucleic acids (PNA), N3'-P5' phosphoamidates (NP), 2' fluoroarabino nucleic acids (FANA), locked nucleic acids (LNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, block-copolymers or gapmers or in other arrangements.

In addition to the aforementioned oligonucleotides, protein expression can also be inhibited using double stranded RNA molecules containing the complementary sequence motifs. Such RNA molecules are known as siRNA molecules in the art (e.g., WO 99/32619 or WO 02/055693). Other siRNAs comprise single stranded siRNAs or double stranded siRNAs having one noncontinuous strand. Again, various chemistries were adapted to this class of oligonucleotides. Also, DNA / RNA hybrid systems are known in the art.

In another embodiment of the present invention, decoy oligonucleotides can be used. These double stranded DNA molecules and chemical modifications thereof do not target nucleic acids but transcription factors. This means that decoy oligonucleotides bind sequence-specific DNA-binding proteins and interfere with the transcription (e.g. Cho-Chung, et al. in Curr. Opin. Mol. Ther., 1999).

In a further embodiment of the invention oligonucleotides that may influence transcription by hybridizing under physiological conditions to the promoter region of a gene may be used. Again various chemistries may adapt to this class of oligonucleotides.

In a further alternative of the invention, DNAzymes may be used. DNAzymes are single-stranded oligonucleotides and chemical modifications therof with enzymatic activity. Typical DNAzymes, known as the "10-23" model, are capable of cleaving single-stranded RNA at specific sites under physiological conditions. The 10-23 model of DNAzymes has a catalytic domain of 15 highly conserved deoxyribonucleotides, flanked by 2 substrate-recognition domains complementary to a target sequence on the RNA. Cleavage of the target mRNAs may result in their destruction and the DNAzymes recycle and cleave multiple substrates.

In another embodiment of the invention, ribozymes can be used. Ribozymes are single-stranded oligoribonucleotides and chemical modifications thereof with enzymatic activity. They can be operationally divided into two components, a conserved stem-loop structure forming the catalytic core and flanking sequences which are reverse complementary to sequences surrounding the target site in a given RNA transcript. Flanking sequences may confer specificity and may generally constitute 14-16 nt in total, extending on both sides of the target site selected.

In a further embodiment of the invention aptamers may be used to target proteins. Aptamers are macromolecules composed of nucleic acids, such as RNA or DNA, and chemical modifications thereof that bind tightly to a specific molecular target and are typically 15-60 nt long. The chain of nucleotides may form intramolecular interactions that fold the molecule into a complex three-dimensional shape. The shape of the aptamer allows it to bind tightly against the surface of its target molecule including but not limited to acidic proteins, basic proteins, membrane proteins, transcription factors and enzymes. Binding of aptamer molecules may influence the function of a target molecule.

All of the above-mentioned oligonucleotides may vary in length between as little as 5, preferably between 8 and 50 nucleotides or nucleobases. More specifically, the oligonucleotides may be antisense oligonucleotides of 8 to 50 nucleotides length that catalyze RNAseH mediated degradation of their target sequence or block translation or re-direct splicing or act as antogomirs; they may be siRNAs of 15 to 30 basepairs length; they may further represent decoy oligonucleotides of 15 to 30 basepairs length; can be complementary oligonucleotides influencing the transcription of genomic DNA of 15 to 30 nucleotides length; they might further represent DNAzymes of 25 to 50 nucleotides length or ribozymes of 25 to 50 nucleotides length or aptamers of 15 to 60 nucleotides length. Such subclasses of oligonucleotides are often functionally defined and can be identical or different or share some, but not all features of their chemical nature or architecture without substantially affecting the teachings of this invention, which focuses on assemblies between TEE's s and oligonucleotides that have a specific advantage in membrane permeability.

The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one or more mismatches within the said continuous stretch of base pairs, although this is less preferred. In general the type and chemical composition of such nucleic acids is of little impact for the performance of the invention and the skilled artisan may find other types of oligonucleotides or nucleic acids suitable for use with this invention.

The assemblies of the present invention between oligonucleotides or other polar molecules and TEE's may further benefit from association or conjugation to ligands. Such ligands may change the biodistribution of the material, in particular after systemic administration of the assemblies. Ligands may also enhance the cellular uptake of the assemblies in that they facilitate entering the endosomal pathway. The skilled artisan is aware of a number of different strategies to incorporate ligands into biomolecular assemblies and examples include, but are not limited to precipitation of oligonucleotides with polycations and targeting antibodies as reported by Song et al. in Nat. Biotechnol. (2005); 23(6):709-17, continuous integration of a targeting aptamers with an active siRNA as reported by Chu et al. (2006) in Nucleic Acid Res. 34(10):e73, direct modification with LDL-targeting alkyl groups as reported by Wolfrum (2007, supra).

Also described herein are pharmaceutical compositions comprising assemblies of nucleic acids with pH-responsive transfection enhancer elements (TEE's). The pharmaceutical composition may be formulated in a suitable pharmacologically acceptable vehicle. Vehicles such as water, saline, phosphate buffered saline are well known to those skilled in the art for this purpose.

In some embodiments said pharmaceutical compositions may be used for the treatment or prophylaxis of inflammatory, immune or autoimmune disorders and/or cancer of humans or non-human animals.

Further described is a pharmaceutical composition for use in methods for the treatment of human or non-human animals in which said pharmaceutical composition comprising conjugates of nucleic acids with transfection enhancer elements is targeted to a specific organ or organs, tumours or sites of infection or inflammation.

In the drawings:

Figure 1 shows the logD response of compound 11-14

Figure 2 shows the relation between logD and pH for different hydrophiles in TEE structures. All hydrophilic elements are located at the terminal position of the alkyl chain. LogD starts to decrease at pH values roughly equal to pKa, such decrease being limited by ion pair formation at physiological ionic strenght.

Figure 3 shows standardized curves for logD wherein pH-pKa is used as x-axis for different hydrophiles in TEE structures. All hydrophilic elements are located at the terminal position of the alkyl chain. 3-cholor octanoic acid is identical with octanoic acid and not shown in the plot.

Figure 4 shows an analysis of the logD vs. pH for monomers, dimers and oligomers of decanoic acid. Curves are in the same order as in the figure legend. It becomes apparent from the graph that monomers or dimers respond to a wider range of pH values with changes in the logD than this is true for the oligomers. Although the left flank of the curve remains essentially unchanged, there is a substantial compression on the right flank, rendering higher oligomers active in a narrow range of pH values.

Figure 5 shows the impact of alkyl chain length in the TEE for different nucleobases and phosphodeoxyribose (mononucleotide form, phosphate terminated with methyl as in compound 22). All species analyzed respond to acidification of the medium with an increase of the logD values of about 2.3 units. Nucleotides modified with TEE's longer than 8 carbon atoms become hydrophobic at pH 4.5. Such shift dramatically ameliorates the hydrophobicity of the oligonucleotide and facilitates transfection of the entire structure.

Figure 6 shows the impact of the addition of TEE's to oligophosphoribose of different length. (A) Oligophosphoriboses having 1, 2, 3 or 4 repetitive units were analyzed towards their logD profile. Each phosphoribose unit contributes a log D of about -3.3units, making it impossible for longer oligonucleotides to cross biological membranes. Also, compounds with a phosphate backbone not modified with a TEE do not response to changes in the pH between pH 3 and pH 10.
(B) Oligophosphoriboses having 1, 2, 3 or 4 repetitive units and modified with TEE's comprising octylcarboxylic groups were analyzed towards their logD profile. The resulting conjugates are less hydrophilic at neutral pH and each unit now contributes only 2.2 units of logD. More importantly, the TEE modified oligophosphoribose responds strongly towards an acidification of the medium and a compound with very little hydrophilic character results. Also, chain extension do no longer contribute to the hydrophilic character, as the impact on logD at pH 4.5 is only 0.25 units per extra unit of the oligomer.

The following examples should further illustrate and enable the invention.

Example 1

Synthesis of N-(3-Amino-propyl)-N'-[3-(4-{3-[4-(3-aminopropylamino)-butylamino]-propylamino}-butylamino)-propyl] -butane-1,4-diamine (compound 4)

A reaction scheme for the synthesis of compound 4 can be found in figure 7.

Step a: Synthesis of {4-[(3-Amino-propyl)-tert-butoxycarbonylamino]-butyl}-(3-tert-butoxycarbonylaminopropyl)-carbamic acid tert-butyl ester (compound 6)

The compound was synthesized according to Geall et al., Chem. Commun. 1998, 2035. Briefly, 10.12 g spermine and 150 ml methanol were stirred and cooled down to -75 °C. Then 5.95 ml trifluoro acetic acid ethylester (99%) were added dropwise. The temperature was raised to 0°C, 42.8 ml di-tert-butyldicarbonate (BOC20) were added and the reaction was stirred at room temperature overnight. About 50 ml of the solvent was removed by rotary evaporation, replaced with 50 ml $H_2O$ and extracted three times with 200 ml diethylether. The organic phase was dried over $Na_2SO_4$, filtered and evaporated under vacuum; the crude product, a colourless oil, was purified by column chromatography on silica gel (eluent 1: dichloromethane / methanol / $NH_4OH$ (25%) 70:10:1; eluent 2: dichloromethane / methanol / $NH_4OH$ (25%) 50:10:1). The product was characterized by $^1$H-NMR and LC-MS.

Step b: Synthesis of Succinic acid bis-(4-nitro-phenyl) ester (compound 10)

36.3 g DCC (N,N'-dicyclohxylcarbodiimide), 0.2 g 4-dimethylaminopyridine, 46.7 g p-nitrophenol, and 16 g succinic acid anhydride and 600 ml acetic acid ethylester were mixed in a round bottom flask and stirred at room temperature for three days. The solvent was removed by rotary evaporation and the yellow residue was recrystallized from 150 ml chloroform. The white product was washed with chloroform, dried and characterized by thin layer chromatography and $^1$H-NMR.

Step c: Synthesis of compound 11

Compound 11 was synthesized according to Graminski et al., Bioorg. Med. Chem. Lett. 2002, 35-40. Briefly, 8.8 g of compound 6 was dissolved in 100 ml dimethylformamide. Then 1.95 N-methyl morpholine and 2.84 g of compound 10 were added and the mixture allowed to stir at room temperature overnight. The solvent was removed by rotary

evaporation and the crude product, a yellow oil, was purified by column chromatography on silica gel (eluent: acetic acid ethylester / methanol 9:1). The solvent was removed and the residue dissolved in 100 ml acetic acid ethylester and 100 ml petrolether. The mixture was washed three times with 150 ml $H_2O$ and the organic phases were dried over $Na_2SO_4$. After a filtration the solvent was removed by rotary evaporation and product characterized by thin layer chromatography, LC-MS and 1H-NMR.

Step d: Synthesis of compound 12

Under $N_2$ atmosphere 4.68 g of compound 11 were dissolved in 40 ml tetrahydrofurane and the reaction mixture was cooled with an ice bath. Then 10.8 ml of a borane-dimethylsulfid-complex (2M in tetrahydrofurane) were added and the reaction was allowed to stir overnight at room temperature. After the addition of 100 ml petrolether, 20 ml acetic acid ethylester and 2ml methanol the solution was chromatographed on a silica gel column (flash chromatography; eluent: acetic acid ethylester). The solvent was removed and the crude product, a colourless oil, was purified by a further column chromatography on silica gel (eluent: acetic acid ethylester). The product was characterized by LC-MS and $^1$H-NMR.

Step e: Synthesis of compound 4

0.92 g of compound 12 was dissolved in 10 ml methanol. The solution was refluxed before a mixture of 15 ml HCl (37%) and 20 ml HCl (2N) was added drop wise. The reaction mixture was refluxed overnight. After the addition of 10 ml H2O the mixture was extracted two times with 50 ml dichloromethane. The solvent of the aqueous phase was removed and the product was characterized by $^1$H-NMR and LC-MS.

**Derivatization of compound 4 with TEE's**

Samples of 50mg (0,1mmol) of compound 4 were alkylated with w-bromo-a-carboxylic acids. Briefly, 0,3mmol or 0,6mmol of either 6-bromohexanoic acid or 10-bromodecanoic acid or an equimolar mixture between the two were dissolved in 2mL dry DMF and 0,1mmol of compound 4 was added to each reaction. The mixture was incubated at 50°C over night, cooled to room temperature and the degree of alkylation was determined by mass spectroscopy.

## Claims

1. Complex comprising a nucleic acid and a carrier system having grafted thereon one or more transfection enhancer elements (TEE's) according to the general formula (I)

   hydrophobic moiety - pH-responsive hydrophilic moiety (I)

   wherein said pH-responsive hydrophilic moiety of said TEE's is a carboxylic acid group and said hydrophobic moiety is a linear, branched or cyclic chain having between 6 and 40 carbon atoms, and
   wherein said carrier system is a polyamine selected from the group of spermine, dispermine, trispermine, tetraspermine, oligospermine, thermine, spermidine, dispermidine, trispermidine, oligospermidine, putrescine, or a polyethylenimine of branched or linear type.

2. The complex according to claim 1, wherein said hydrophobic moiety has between 6 and 20 carbon atoms.

3. The complex according to claim 1, wherein said hydrophobic moiety is a straight alkyl chain.

4. The complex according to claim 1, wherein said nucleic acid is an oligonucleotide.

5. The complex according to claim 1, wherein said nucleic acid is a polynucleotide or a DNA plasmid.

6. Polycation having grafted thereon TEE's (transfection enhancer elements) of the following general formula (I):

   hydrophobic moiety - pH-responsive hydrophilic moiety (I)

   wherein said pH-responsive hydrophilic moiety is a carboxylic acid group and said hydrophobic moiety is a linear, branched or cyclic chain with a length of between 6 and 40 carbon atoms, and
   wherein said polycation is a polyamine selected from the group of polyethylenimine, spermine, dispermine, trispermine, tetraspermine, oligospermine, thermine, spermidine, or putrescine.

7. The polycation according to claim 6, wherein the polycations comprise more than 4 and less than 1000 charged elements.

8. Nucleic acid comprising a nucleic acid moiety and two or more transfection enhancer elements (TEE's) according to the general formula (I)

hydrophobic moiety - pH-responsive hydrophilic moiety (I)

wherein said pH sensitive hydrophilic moiety of said TEE's is a carboxyl group; said hydrophobic moiety is a linear, branched or cyclic chain having between 6 and 40 carbon atoms; and
wherein said nucleic acid comprises polymeric or oligomeric extensions that carry one or more of said TEEs, said polymeric or oligomeric extensions being polyamines selected from the group of spermine, dispermine, trispermine, tetraspermine, oligospermine, thermine, spermidine, dispermidine, trispermidine, oligospermidine, putrescine, a polyethylenimine of branched or linear type, or polyallylamine.

9. The nucleic acid according to claim 8, wherein said polyamine is a linear polyamine having between 6 and 12 charged groups, these charged groups being separated by 3 to 6 bonds.

**Patentansprüche**

1. Komplex umfassend eine Nukleinsäure und ein Trägersystem, das ein oder mehrere Transfektionsverstärkerelemente (TEEs) gemäß der allgemeinen Formel (I) aufgepfropft aufweist:

hydrophobe Gruppe - pH-reagierende hydrophile Gruppe (I)

worin die pH-reagierende hydrophile Gruppe der TEEs eine Carbonsäuregruppe ist und die hydrophobe Gruppe eine lineare, verzweigte oder cyclische Kette mit zwischen 6 und 40 Kohlenstoffatomen ist, und
worin das Trägersystem ein Polyamin ausgewählt aus der Gruppe Spermin, Dispermin, Trispermin, Tetraspermin, Oligospermin, Thermin, Spermidin, Dispermidin, Trispermidin, Oligospermidin, Putrescin oder ein Polyethylenimin vom verzweigten oder linearen Typ ist.

2. Der Komplex gemäß Anspruch 1, worin die hydrophobe Gruppe zwischen 6 und 20 Kohlenstoffatome aufweist.

3. Der Komplex gemäß Anspruch 1, worin die hydrophobe Gruppe eine lineare Alkylkette ist.

4. Der Komplex gemäß Anspruch 1, worin die Nukleinsäure ein Oligonucleotid ist.

5. Der Komplex gemäß Anspruch 1, worin die Nukleinsäure ein Polynucleotid oder ein DNA Plasmid ist.

6. Polycation, das TEEs (Transfektionsverstärkerelemente) gemäß der folgenden allgemeinen Formel (I) aufgepfropft aufweist:

hydrophobe Gruppe - pH-reagierende hydrophile Gruppe (I)

worin die pH-reagierende hydrophile Gruppe der TEEs eine Carbonsäuregruppe ist und die hydrophobe Gruppe eine lineare, verzweigte oder cyclische Kette mit einer Länge zwischen 6 und 40 Kohlenstoffatomen ist, und
worin das Polycation ein Polyamin ausgewählt aus der Gruppe Polyethylenimin, Spermin, Dispermin, Trispermin, Tetraspermin, Oligospermin, Thermin, Spermidin, oder Putrescin ist.

7. Das Polycation gemäß Anspruch 6, worin die Polycatione mehr als 4 und weniger als 1000 geladene Elemente aufweisen.

8. Nukleinsäure umfassend eine Nukleinsäuregruppe und zwei oder mehr Transfektionsverstärkerelemente (TEEs) gemäß der allgemeinen Formel (I):

hydrophobe Gruppe - pH-reagierende hydrophile Gruppe (I)

worin die pH-reagierende hydrophile Gruppe der TEEs eine Carbonsäuregruppe ist; die hydrophobe Gruppe eine lineare, verzweigte oder cyclische Kette mit zwischen 6 und 40 Kohlenstoffatomen ist, und

worin die Nukleinsäure polymere oder oligomere Extensionen aufweist, die ein oder mehrere der TEEs tragen, wobei die polymeren oder oligomeren Extensionen Polyamine sind, ausgewählt aus der Gruppe Spermin, Dispermin, Trispermin, Tetraspermin, Oligospermin, Thermin, Spermidin, Dispermidin, Trispermidin, Oligospermidin, Putrescin, ein Polyethylenimin vom verzweigten oder linearen Typ oder Polyallylamin.

9. Die Nukleinsäure gemäß Anspruch 8, worin das Polyamin ein lineares Polyamin mit zwischen 6 und 12 geladenen Gruppen ist, wobei die geladenen Gruppen durch 3 bis 6 Bindungen getrennt sind.

**Revendications**

1. Complexe comprenant un acide nucléique et un système de support sur lequel est/sont greffé(s) un ou plusieurs élément(s) amplificateur(s) de transfection (TEE) de formule générale (I)

fragment hydrophobe - fragment hydrophile sensible au pH (I)

dans lequel ledit fragment hydrophile sensible au pH desdits TEE est un groupe acide carboxylique et ledit fragment hydrophobe est une chaîne linéaire, ramifiée ou cyclique comportant entre 6 et 40 atomes de carbone, et dans lequel ledit système de support est une polyamine choisie dans l'ensemble comprenant la spermine, la dispermine, la trispermine, la tétraspermine, l'oligospermine, la thermine, la spermidine, la dispermidine, la trispermidine, l'oligospermidine, la putrescine, et une polyéthylène-imine de type ramifié ou linéaire.

2. Complexe selon la revendication 1, dans lequel ledit fragment hydrophobe comporte entre 6 et 20 atomes de carbone.

3. Complexe selon la revendication 1, dans lequel ledit fragment hydrophobe est une chaîne droite alkyle.

4. Complexe selon la revendication 1, dans lequel ledit acide nucléique est un oligonucléotide.

5. Complexe selon la revendication 1, dans lequel ledit acide nucléique est un polynucléotide ou un ADN plasmidique.

6. Polycation sur lequel sont greffés des TEE (éléments amplificateurs de transfection) de formule générale (I) suivante :

fragment hydrophobe - fragment hydrophile sensible au pH (I)

dans lequel ledit fragment hydrophile sensible au pH est un groupe acide carboxylique et ledit fragment hydrophobe est une chaîne linéaire, ramifiée ou cyclique présentant une longueur comprise entre 6 et 40 atomes de carbone, et lequel polycation est une polyamine choisie dans l'ensemble comprenant la polyéthylène-imine, la spermine, la dispermine, la trispermine, la tétraspermine, l'oligospermine, la thermine, la spermidine, et la putrescine.

7. Polycation selon la revendication 6, lequel polycation comprend plus de 4 et moins de 1000 éléments chargés.

8. Acide nucléique comprenant un fragment d'acide nucléique et deux ou plus de deux éléments amplificateurs de transfection (TEE) de formule générale (I)

fragment hydrophobe - fragment hydrophile sensible au pH (I)

dans lequel ledit fragment hydrophile sensible au pH desdits TEE est un groupe acide carboxylique ; ledit fragment hydrophobe est une chaîne linéaire, ramifiée ou cyclique comportant entre 6 et 40 atomes de carbone, et dans lequel ledit acide nucléique comprend des extensions polymères ou oligomères qui portent un ou plusieurs dudit/desdits TEE, lesdites extensions polymères ou oligomères étant des polyamines choisies dans l'ensemble comprenant la spermine, la dispermine, la trispermine, la tétraspermine, l'oligospermine, la thermine, la spermidine, la dispermidine, la trispermidine, l'oligospermidine, la putrescine, une polyéthylène-imine de type ramifié ou linéaire, et la polyallylamine.

9. Acide nucléique selon la revendication 8, dans lequel ladite polyamine est une polyamine linéaire comportant entre 6 et 12 groupes chargés, ces groupes chargés étant séparés par 3 à 6 liaisons.

Figure 1:

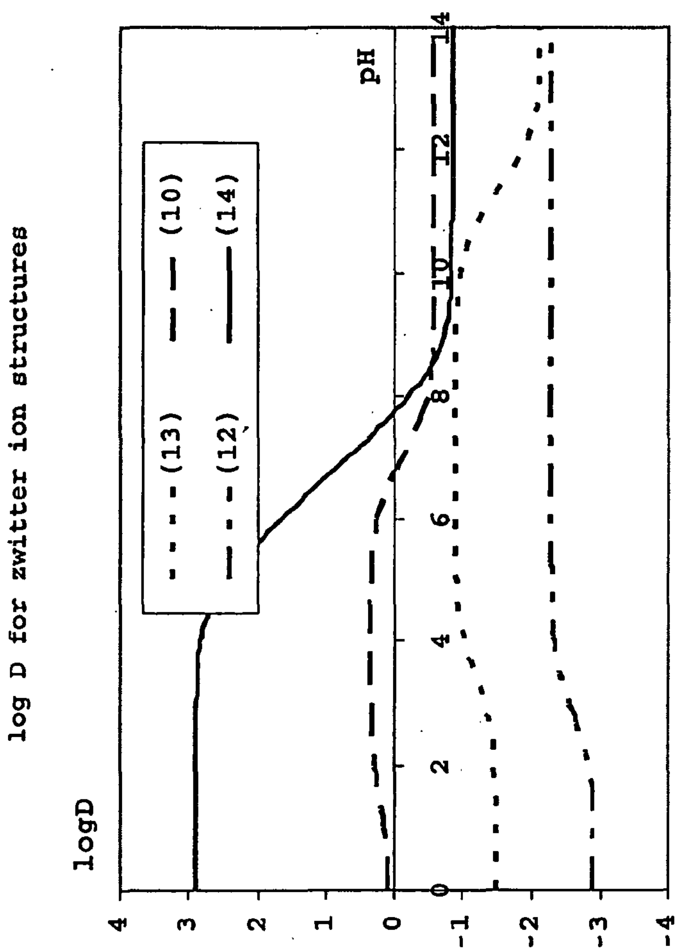
log D for zwitter ion structures
logD
pH
(13)
(10)
(12)
(14)
0
2
4
6
8
10
12
14
4
3
2
1
0
-1
-2
-3
-4

Figure 2:

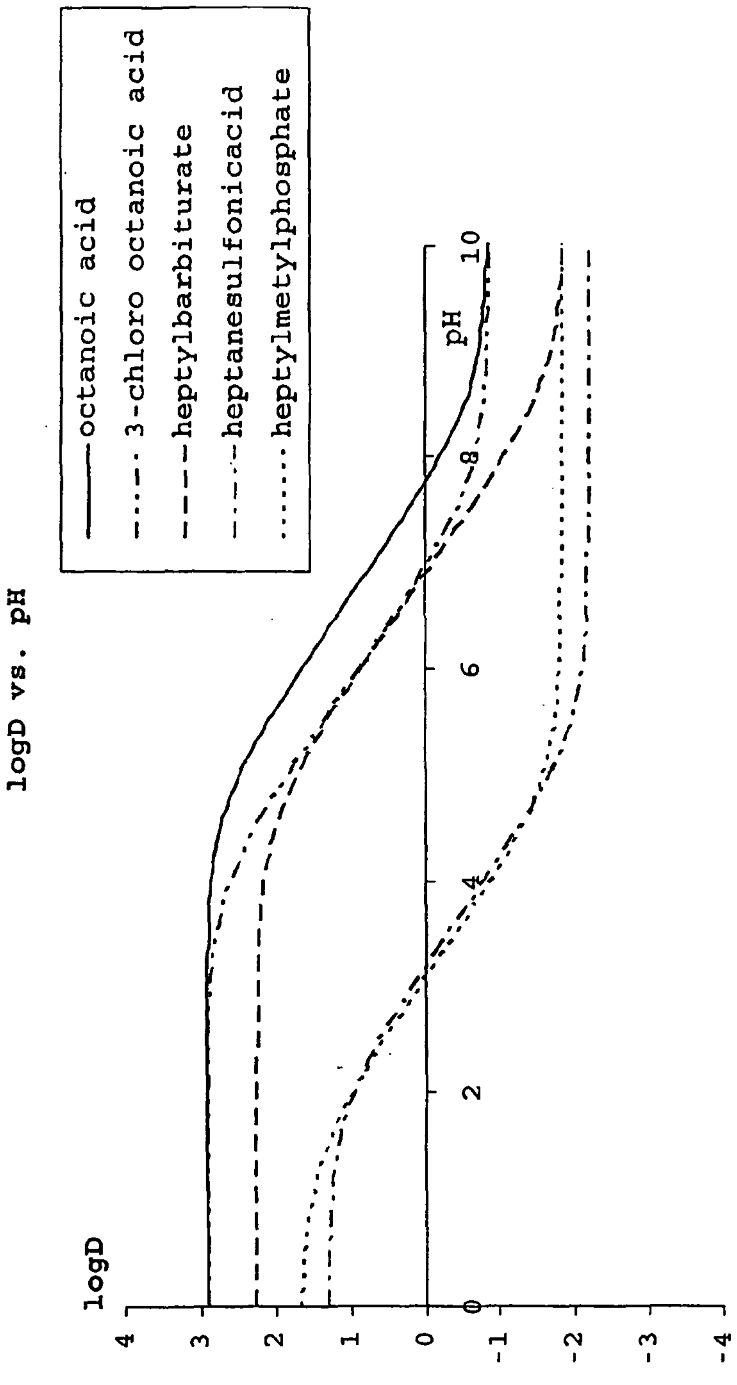
logD vs. pH
octanoic acid
3-chloro octanoic acid
heptylbarbiturate
heptanesulfonicacid
heptylmetylphosphate
logD
pH
4
3
2
1
0
-1
-2
-3
-4
0
2
4
6
8
10

Figure 3:

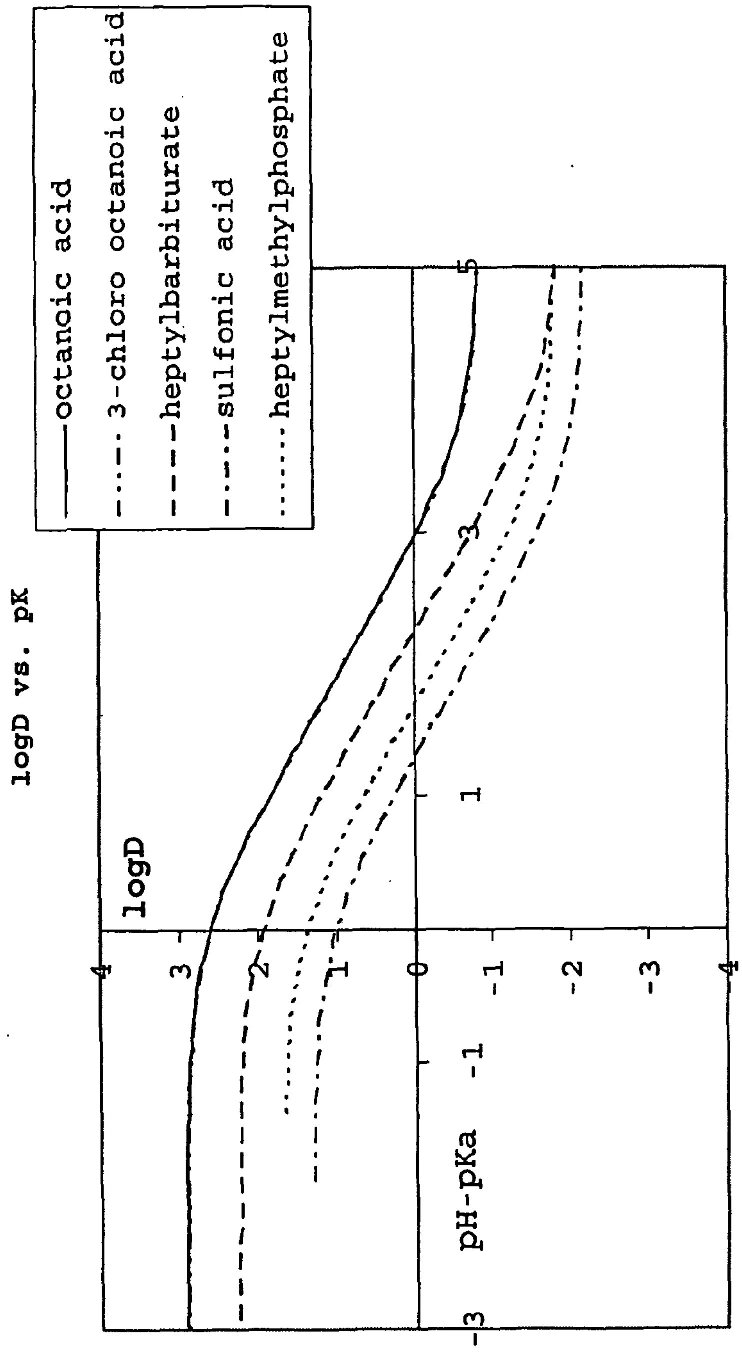
logD vs. pK
octanoic acid
3-chloro octanoic acid
heptylbarbiturate
sulfonic acid
heptylmethylphosphate
logD
pH-pKa
4
3
2
1
0
-1
-2
-3
-4
-3
-1
1
3
5

Figure 4:

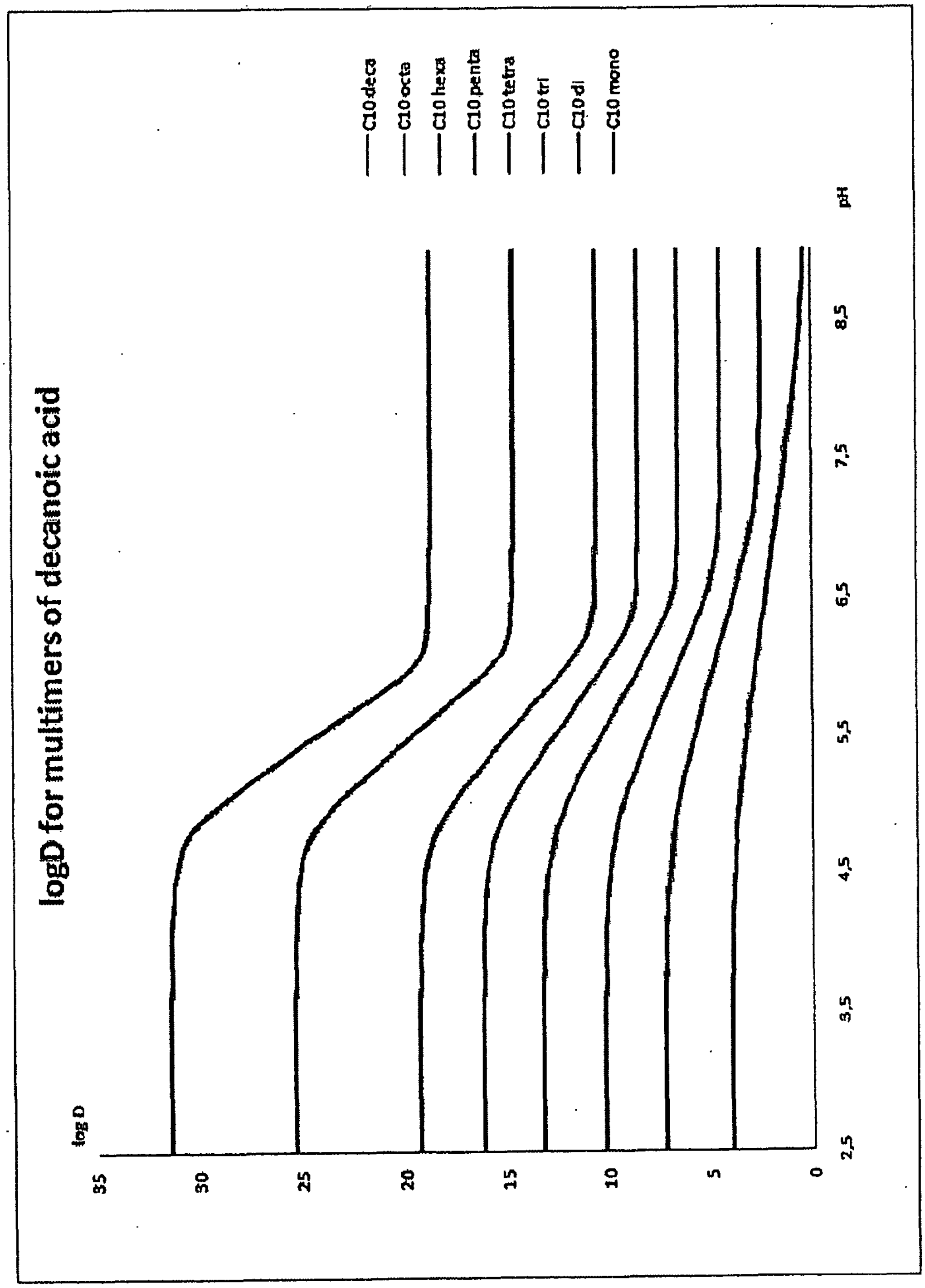
logD for multimers of decanoic acid
log D
35
30
25
20
15
10
5
0
2,5
3,5
4,5
5,5
6,5
7,5
8,5
pH
C10 deca
C10 octa
C10 hexa
C10 penta
C10 tetra
C10 tri
C10 di
C10 mono

Figure 5:

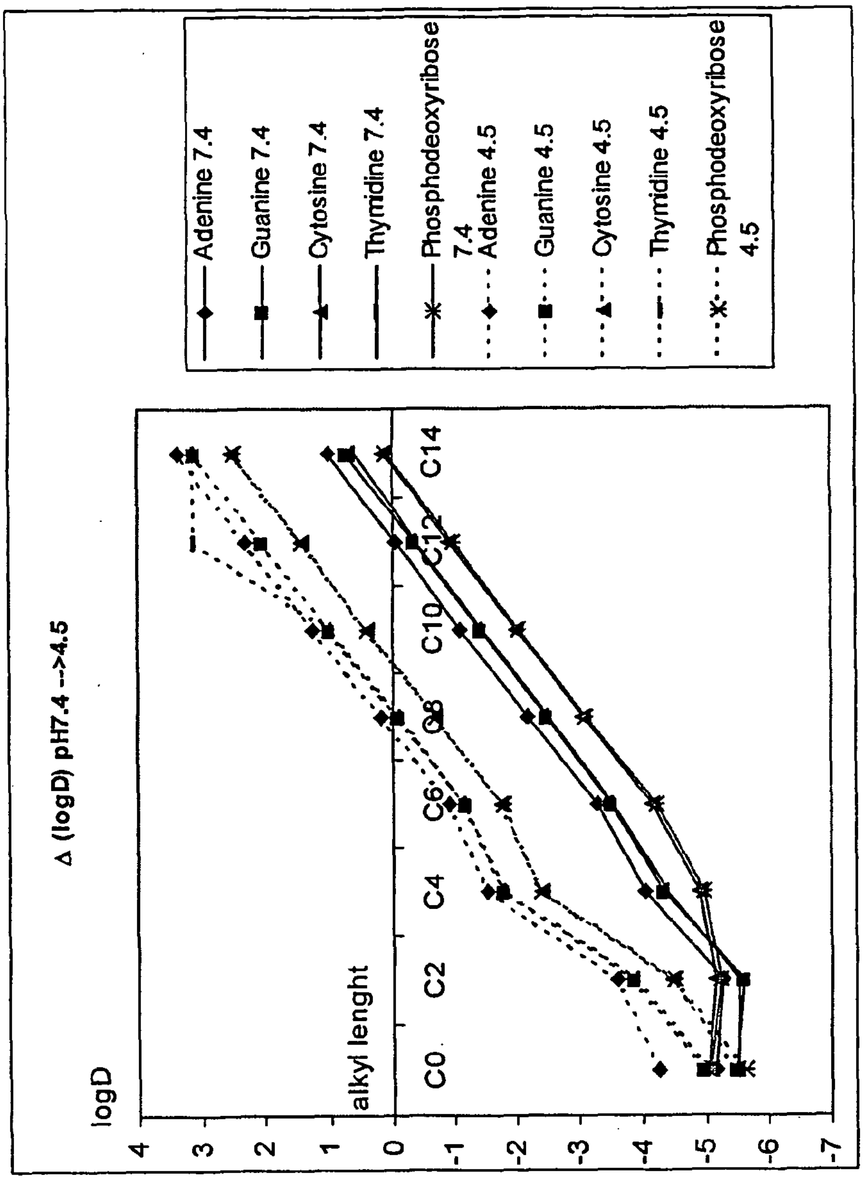
Δ (logD) pH7.4 -->4.5
logD
alkyl lenght
4
3
2
1
0
-1
-2
-3
-4
-5
-6
-7
C0
C2
C4
C6
C8
C10
C12
C14
Adenine 7.4
Guanine 7.4
Cytosine 7.4
Thymidine 7.4
Phosphodeoxyribose 7.4
Adenine 4.5
Guanine 4.5
Cytosine 4.5
Thymidine 4.5
Phosphodeoxyribose 4.5

Figure 6:

(A)

(B)

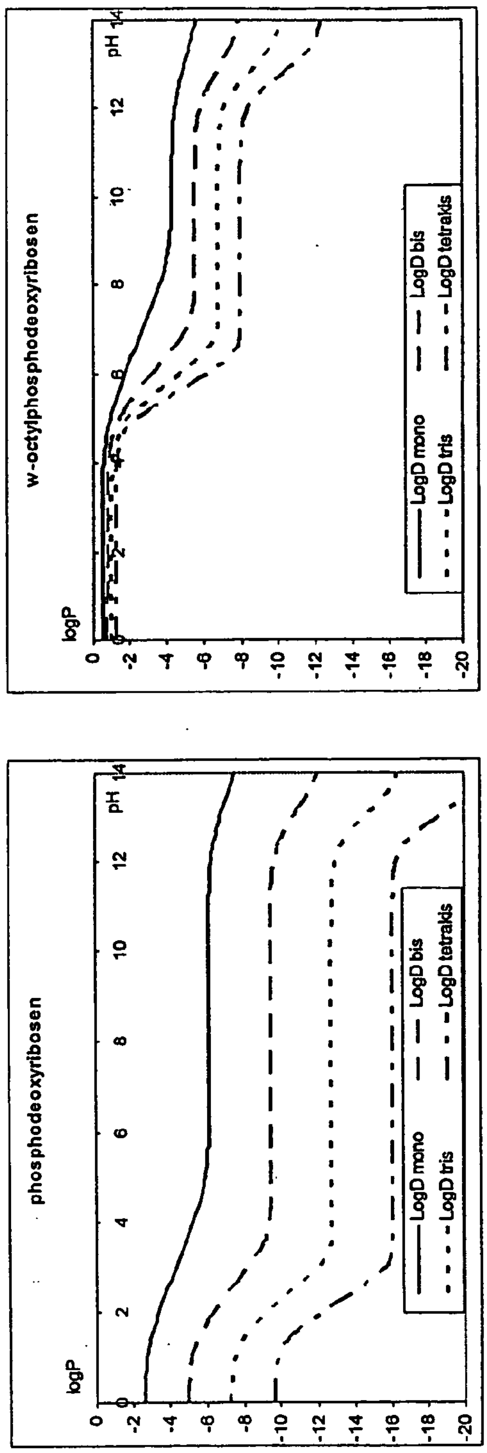
phosphodeoxyribosen
w-octylphosphodeoxyribosen
logP
pH
LogD mono
LogD bis
LogD tris
LogD tetrakis
0
-2
-4
-6
-8
-10
-12
-14
-16
-18
-20
2
4
6
8
10
12
14

Figure 7:

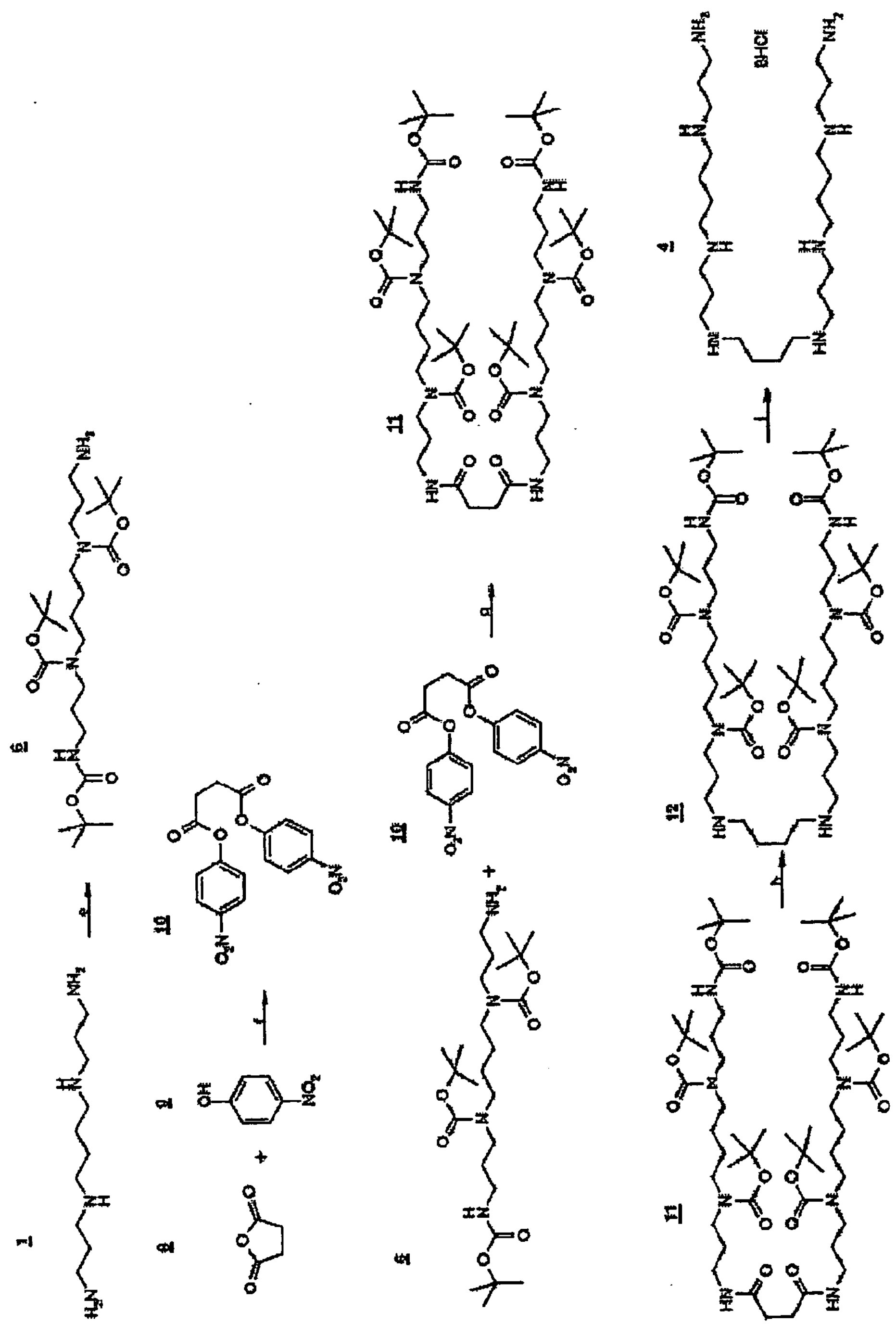

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6693187 B
- US 7067641 B
- US 20040116687 A
- US 20060293511 A
- US 4958013 A, Letsinger
- US 6153737 A, Manoharan
- US 6753423 B
- US 6989434 B1
- US 4458066 A
- US 6277967 B, Guzaev and Manoharan
- US 6919437 B, Manoharan
- US 6320041 B
- WO 9821322 A
- DE 19753182
- EP 1392341 A
- WO 9932619 A
- WO 02055693 A


### Non-patent literature cited in the description


- **LIPINSKI et al.** *Advanced Drug Delivery Reviews,* 1997, vol. 23, 3-25
- **MADDEN et al.** *Chem. Phys. Lipids,* 1990, vol. 53 (1), 37-46
- **HARRIGAN et al.** *Biochim. Biophys. Acta,* 1993, vol. 1149 (2), 329-338
- **CLERCS et al.** *Biochim. Biophys. Acta,* 1995, vol. 1240 (2), 257-265
- **MILLER ; TS'O.** *Annu Rep Med Chem,* 1981, vol. 23, 295
- **ECKSTEIN.** *Trends Biochem Sci,* 1989, vol. 14, 97
- **NIELSEN.** *Tetrahedron Lett,* 1988, vol. 29, 2911
- **RUDOLPH et al.** *Nucleosides and Nucleotides,* 1996, vol. 15, 1725
- **SHEEHAN et al.** *Nucl Acid Res,* 2003, vol. 31, 4109-4118
- *Proc. Natl. Acad. Sci.,* 1989, vol. 86, 6553-6556
- **SOUTSCHEK et al.** *Nature,* 2004, vol. 432 (7014), 173-178
- **WOLFRUM et al.** *Nat Biotech,* 2007, vol. 25, 1149-1157
- **NARANG et al.** *Meth. Enzymol.,* 1979, vol. 68, 90-99
- **BROWN et al.** *Method Enzymol.,* 1979, vol. 68, 109-151
- **BEAUCAGE et al.** *Tetrahedron Lett.,* 1981, vol. 22, 1859-1862
- **MATTEUCCI et al.** *J. Am. Chem. Soc.,* 1981, vol. 103, 3185-3191
- **SHEEHAN.** *Nuc Acid Res,* 2003, vol. 31, 4109-4118
- **BOUSSIF et al.** *Proc. Natl. Acad. Sci.,* 1995, vol. 92 (16), 7297-7301
- **GODBEY et al.** *J. Control. Release,* 1999, vol. 60 (2-3), 149-160
- **JANES et al.** *Adv. Drug Deliv. Rev.,* 2001, vol. 47 (1), 83-97
- **HU-LIESKOVAN et al.** *Cancer Res.,* 2005, vol. 65 (19), 8984-8992
- **KAUL et al.** *Pharm. Res.,* 2005, vol. 22 (6), 951-961
- **SANO et al.** *Adv. Drug Deliv. Rev.,* 2003, vol. 55 (12), 1651-1677
- **G. T. HERMANSON.** Bioconjugate Techniques. Academic Press, 1996
- **PRAKASH ; BHAT.** *Curr Top Med Chem,* 2007, vol. 7, 641-649
- **CHO-CHUNG et al.** *Curr. Opin. Mol. Ther.,* 1999
- **SONG et al.** *Nat. Biotechnol.,* 2005, vol. 23 (6), 709-17
- **CHU et al.** *Nucleic Acid Res.,* 2006, vol. 34 (10), e73
- **GEALL et al.** *Chem. Commun.,* 1998, 2035
- **GRAMINSKI et al.** *Bioorg. Med. Chem. Lett.,* 2002, 35-40